(19) Europäisches Patentamt
European Patent Office
Office européen des brevets

(11) EP 3 558 940 B1

(12) # EUROPÄISCHE PATENTSCHRIFT

(45) Veröffentlichungstag und Bekanntmachung des Hinweises auf die Patenterteilung:
**20.01.2021 Patentblatt 2021/03**

(21) Anmeldenummer: **17826205.1**

(22) Anmeldetag: **18.12.2017**

(51) Int Cl.:
***C07C 263/10*** *(2006.01)* ***C07C 263/20*** *(2006.01)*

(86) Internationale Anmeldenummer:
**PCT/EP2017/083379**

(87) Internationale Veröffentlichungsnummer:
**WO 2018/114846 (28.06.2018 Gazette 2018/26)**

(54) **VERFAHREN ZUR HERSTELLUNG EINES ISOCYANATS**

METHOD FOR DRYING OF TDI REMNANTS

PROCÉDÉ DE SÉCHAGE DE RÉSIDUS DE TDI

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2016 EP 16205620**

(43) Veröffentlichungstag der Anmeldung:
**30.10.2019 Patentblatt 2019/44**

(73) Patentinhaber: **Covestro Intellectual Property GmbH & Co. KG**
**51373 Leverkusen (DE)**

(72) Erfinder:
- **LODDENKEMPER, Tim**
**41542 Dormagen (DE)**
- **ARRAS, Jürgen**
**25524 Itzehoe (DE)**
- **DUGAL, Markus**
**47906 Kempen (DE)**

(74) Vertreter: **Levpat**
**c/o Covestro AG**
**Gebäude 4825**
**51365 Leverkusen (DE)**

(56) Entgegenhaltungen:
**DD-A5- 288 599**
**DE-A1- 4 104 305**
**US-A- 2 884 360**
**US-A- 2 884 362**
**US-A- 3 987 075**
**US-A- 4 372 891**
**US-A- 4 918 220**
**US-A- 5 314 588**
**US-B1- 6 307 096**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Bei der großtechnischen Herstellung von Isocyanaten fallen Destillationssumpfströme an, die der weiteren Aufarbeitung bedürfen. Diese Destillationssumpfströme enthalten neben einem aus nur sehr schwer oder überhaupt nicht unzersetzt verdampfbaren Verbindungen bestehenden Destillationsrückstand (auch kurz als Rückstand bezeichnet) auch noch Anteile an dem angestrebten Zielprodukt (d. i. das herzustellende Isocyanat). Die vorliegende Erfindung betrifft ein Verfahren, das es ermöglicht, diesen Anteil an herzustellendem Isocyanat in einem Destillationssumpfstrom, welcher bei der Aufarbeitung des in einem Isocyanat-Herstellungsprozess gebildeten rohen, flüssigen, das herzustellende Isocyanat enthaltenden Verfahrensprodukts anfällt, auf effiziente Art zurückzugewinnen. Insbesondere betrifft die vorliegende Erfindung einen Trocknungsschritt, in welchem das herzustellende Isocyanat unter Ausbildung eines von diesem Isocyanat weitgehend bis vollständig befreiten Feststoffes zurückgewonnen wird. Dieser Trocknungsschritt ist durch einen Mindestgehalt an Carbodiimidgruppen-haltigen Verbindungen von 15 Massen-%, bevorzugt 20 Massen-%, besonders bevorzugt 30 Massen-%, bezogen auf die Gesamtmasse des der eingesetzten Trocknungsvorrichtung zugeführten Destillationsrückstands, gekennzeichnet, wobei dieser Mindestgehalt auch durch eine *In situ*-Carbodiimidisierung eingestellt werden kann.

Der Stand der Technik zur Behandlung der genannten Destillationsrückstände enthaltenden Destillationssumpfströme der Isocyanat-Herstellung beschreibt verschiedene Verfahren. Generelle Ziele der so genannten "Rückstandsbehandlung" sind die Maximierung der Isocyanat-Ausbeute, die Minimierung des Mengenanfalls an von Isocyanat weitgehend bis vollständig befreitem Rückstand, eine betriebssichere Ausgestaltung der Rückstandsbehandlung und eine möglichst sinnvolle kostengünstige und einfache Verwertung der für den Isocyanat-Herstellprozess nicht mehr nutzbaren Rückstandsmenge.

Prinzipiell sind folgende Verfahren bekannt:

Grundsätzlich kann ein Destillationssumpfstrom enthaltend den Destillationsrückstand kontinuierlich oder diskontinuierlich verbrannt werden. Das Verfahren ist technisch einfach und kann zur Nutzdampferzeugung eingesetzt werden, sofern eine dafür geeignete Anlage zur thermischen Verwertung in der Nähe der Isocyanatproduktionsanlage existiert, um eine Entsorgung über eine Rohrleitungsanbindung zu gewährleisten. Der große Nachteil dieses Verfahrens ist jedoch der Ausbeuteverlust, dadurch verursacht, dass der Destillationssumpfstrom enthaltend den Destillationsrückstand immer auch Anteile des Wertprodukts Isocyanat enthält, das mitverbrannt wird. Würde die Destillation des Isocyanats so betrieben, dass das Isocyanat aus dem Destillationssumpf vollständig oder annähernd vollständig entfernt würde, verbliebe ein fester, nur sehr schwer zu verarbeitender Destillationssumpf, der fast ausschließlich aus Rückstand bestünde. Um dies zu vermeiden, werden die Destillationsbedingungen üblicherweise so gewählt, dass das Sumpfprodukt der Destillationskolonne flüssig bleibt, was jedoch nur gelingt, wenn dieses noch substanzielle Anteil des herzustellenden Isocyanats enthält, die somit unweigerlich mit der Verbrennung zugeführt werden.

US 5,314,588 beschreibt ein Verfahren zur Gewinnung eines im Wesentlichen aus Toluylendiisocyanat (fortan auch TDI) bestehenden Destillats und eines Isocyanatgruppen aufweisenden Sumpfprodukts mit einem Gehalt an Toluylendiisocyanat von unter 200 ppm (Gewicht) aus A) Destillationsrückständen der Toluylendiisocyanatherstellung durch Vermischen

- der Destillationsrückstände A) mit
- B) Polyisocyanaten oder Polyisocyanatgemischen der Diphenylmethanreihe mit einem NCO-Gehalt von mindestens 15 Gew.-%,

und destillative Aufarbeitung des Gemischs, dadurch gekennzeichnet, dass man das Gemisch vor oder während der destillativen Aufarbeitung auf Temperaturen von 190 bis 250 °C erhitzt und hierdurch eine weitgehende Substitution des im Destillationsrückstand A) reversibel gebunden Toluylendiisocyanats durch Polyisocyanat B) bewirkt.

US 2,884,360 beschreibt ein Verfahren zur Rückgewinnung organischer Isocyanate aus rohen, die organischen Isocyanate enthaltenden Mischungen durch Destillation der Mischungen in Gegenwart von Dialkyl-, Diaryl- oder Alkylaryl-Sulfonen.

US 2,884,362 beschreibt ein Verfahren zur Rückgewinnung organischer Isocyanate aus rohen, die organischen Isocyanate enthaltenden Mischungen durch Destillation der Mischungen in Gegenwart eines Polyetherpolyols.

US 3,987,075 beschreibt ein Verfahren zur Extraktion eines organischen Isocyanats aus einer nach Lösungsmittelabtrennung erhaltenen rohen Reaktionsmischung der Phosgenierung des korrespondierenden Amins, bei welchem ein Alkyl-substituiertes Benzol mit 6 bis 14 Kohlenstoffatomen in der Alkylgruppe bei einer Temperatur von 130 bis 280 °C verwendet wird,

US 4,372,891 beschreibt ein Verfahren zur Rückgewinnung von TDI aus einem Rückstandsprodukt der der Herstellung des TDI folgenden Destillation, bei welchem das Rückstandsprodukt derart mit einem Lösungsmittel vermischt wird, dass darin enthaltenes TDI in Lösung geht während polymere Anteile des Rückstands als Suspension verbleiben. Als Lösungsmittel eignen sich Gemische aus einem aliphatischen Kohlenwasserstoff mit einer Molmasse im Bereich von der des Hexans bis der des Dodecans und einem aromatischen oder chlorierten Kohlenwasserstoff mit einem ähnlichen Siedpunkt wie der aliphatische Kohlenwasserstoff, wobei das Verhältnis von aliphatischem Kohlenwasserstoff zu aromatischem Kohlenwasserstoff im Bereich von 90 : 10 und 10 : 90 liegt. Das in Lösung gegangene TDI wird durch Filtration von den suspendierten polymeren Bestandteilen getrennt.

DE 4104305 A1 beschreibt ein Verfahren zur Aufarbeitung von Destillationsrückständen der Toluylendiisocyanatherstellung als flüssiger Brennstoff durch Umwandlung des nicht lagerstabilen Rückstandes der Toluylendiisocyanatherstellung in eine über mehr als eine Woche lagerstabile Form, dadurch gekennzeichnet, dass der Destillationsrückstand mit einem aromatischen oder zumindest wesentliche Anteile an Aromaten enthaltenden Lösungsmittel gemischt wird, wobei der Anteil an Lösungsmittel bei 15 kg bis 30 kg auf 100 kg Lösung eingestellt wird.

US 6,307,096 beschreibt ein Verfahren zur Herstellung eines aliphatischen Diisocyanats, umfassend die Schritte des: (a) Phosgenierens eines aliphatischen Diamins in Gegenwart eines inerten Lösungsmittels oder Gases zur Bildung einer rohen Reaktionsmischung; (b) Destillierens der rohen Reaktionsmischung zur Bildung eines Produktionsstroms eines aliphatischen Diisocyanats und eines Abfallstroms eines aliphatischen Diisocyanats; (c) Einführens des Abfallstroms eines aliphatischen Diisocyanats in eine Kammer und das Einwirkenlassen von Bedingungen eines überkritischen Fluids auf den Abfallstrom, die ausreichend sind, um eine beträchtliche Menge der aliphatischen Diisocyanat-Komponente im überkritischen Fluid zu lösen; (d) Trennens der gelösten aliphatischen Diisocyanat-Komponente vom Abfallstrom, wobei es sich beim verbleibenden Abfallstrom um einen überkritisch von aliphatischem Diisocyanat gereinigten Abfallstrom handelt; (e) Senkens des Drucks, das ausreichend ist, um die aliphatische Diisocyanat-Verbindung auszufällen.

US 4,918,220 beschreibt ein Verfahren zur Abtrennung von in den bei seiner Herstellung anfallenden Rückständen enthaltenem TDI. Das Verfahren ist dadurch gekennzeichnet, dass diese Rückstände mit einem inerten Gas im flüssigen oder überkritischen Zustand behandelt werden, gegebenenfalls in Gegenwart eines Coextraktionsmittels ausgewählt aus den Estern, den chlorierten oder nicht chlorierten aromatischen Kohlenwasserstoffen und den aliphatischen Kohlenwasserstoffen. Das Verfahren eignet sich insbesondere zur Behandlung von in Form von Teeren vorliegenden Rückständen.

DD 288599 betrifft ein Reinigungsverfahren organischer Isocyanate von chlorhaltigen Verbindungen. Dabei wird organisches Isocyanat in Gegenwart von 0,1 bis 5 Massen-% Carbodiimiden bei einer Temperatur von 453 bis 513 K einer thermischen Behandlung ausgesetzt. Der gebildete Chlorwasserstoff wird anschließend durch Strippen mit Inertgas entfernt.

Zur Minimierung der Isocyanatausbeuteverluste kann der Destillationssumpfstrom in einen gerührten und beheizten Behälter überführt und mit hochsiedenden Kohlenwasserstoffen, vorzugsweise Bitumen, die unter den Destillationsbedingungen inert sind, vermischt werden, um möglichst vollständig das im Rückstand noch vorhandene freie Isocyanat abzudestillieren **(**EP 0 548 685 A2**).** Der verbleibende, von Isocyanat weitgehend befreite Rückstand kann als rieselfähiger Feststoff ausgetragen und einer Verbrennung zugeführt werden. Nachteile dieses Verfahrens sind neben dem Einsatz eines prozessfremden Stoffes (Bitumen) Ausbeuteverluste durch Polymerisation des Isocyanats, da der Prozess hohe Verweilzeiten bei hoher Temperatur beinhaltet.

Ein weiteres Verfahren zur Rückstandsbehandlung, beschrieben in EP 0 626 368 A1**,** ist gekennzeichnet durch den Einsatz von *beheizten, produktumschaufelnden Vakuumtrocknern mit horizontaler Welle.* Durch Einsatz von zum Beispiel Bitumen wird wie im oben genannten Beispiel des Verfahrens gemäß EP 0 548 685 A2 der verbleibende Rückstand als rieselfähiger Feststoff erhalten, der als Brennstoff zum Beispiel in Zementwerken eingesetzt werden kann. Vorteil dieses Verfahrens gegenüber dem oben genannten ist eine Ausbeuteerhöhung. Der Einsatz mechanisch bewegter Teile birgt jedoch grundsätzlich die Gefahr von erhöhtem Verschleiß und damit einhergehendem Wartungsaufwand. Dies gilt insbesondere, wenn der aufzuarbeitende Rückstand eine sehr hohe Viskosität aufweist. Der Einsatz von derartigen Trocknungsvorrichtungen mit mechanisch bewegten Teilen, obwohl grundsätzlich bewährt, ist daher nicht immer frei von Herausforderungen im betrieblichen Alltag. Die vorliegende Erfindung befasst sich unter anderem mit solchen Herausforderungen.

In der Patentliteratur sind auch Verfahren beschrieben, in denen Isocyanat-Destillationssumpfströme enthaltend Destillationsrückstände chemisch umgesetzt werden, um industriell nutzbare Wertstoffe zu erhalten, wie zum Beispiel die Umsetzung eines Destillationssumpfstroms enthaltend Rückstand aus der Herstellung von Toluylendiisocyanat mit Alkanolamin **(**US 5,902,459**)** oder mit Isocyanaten der Diphenylmethanreihe **(**DE 42 11 774 A1**,** US 3,694,323**).**

Die Hydrolyse von Isocyanat- Destillationssumpfströmen mit Wasser zwecks Rückgewinnung des Ausgangsamins, insbesondere bei der Herstellung von Toluylendiisocyanat (nachfolgend TDI), ist ein bereits seit längerer Zeit bearbeitetes Gebiet und beispielsweise in US 3,128,310**,** US 3,331,876**,** GB 795,639**,** DE 27 03 313 A1 und EP 1935 877 A1 beschrieben. Unbefriedigend bei diesen Verfahren ist, dass ein Teil des Wertproduktes Isocyanat wieder zum Ausgangsstoff hydrolisiert und erneut phosgeniert werden muss. Dadurch wird das im Destillationssumpfstrom enthaltene Isocyanat zwar einer sinnvollen stofflichen Verwertung zugeführt, jedoch wäre es wünschenswert, das Isocyanat

als solches aus dem Destillationssumpfströme zurückgewinnen zu können.

EP 1 413 571 A1 und EP 1 371 633 A1 befassen sich mit der Optimierung der Aufarbeitung von TDI durch Einsatz einer Trennwandkolonne in der Destillation, was unter anderem eine Reduzierung des Gehalts an TDI im Sumpfprodukt zur Folge hat. Aber auch hier kann nicht völlig verhindert werden, dass ein Isocyanat enthaltender Destillationssumpfstrom anfällt.

EP 0 017 972 A1 beschreibt ein Verfahren zur Abtrennung von TDI und/oder höhersiedenden Lösungsmitteln aus Destillationssumpfströmen, die bei der Herstellung von TDI durch Phosgenierung von Toluylendiamin entstehen, durch Eindampfen in einer Wirbelschicht bei Temperaturen von 140 bis 280 °C. Bei diesem Verfahren werden die mittels der Einbringvorrichtung in den Wirbelbehälter eingebrachten Tropfen des Destillationssumpfstromes auf die Oberfläche der Vorlage-Partikel aufgesprüht und spreiten dort, was zur Verdampfung des Wertprodukts (TDI und/oder Lösungsmittel) und zum Aufbau schalenförmiger Granulate aus Wertstoff-freiem Rückstand führt. Ein solcher Granulationsprozess weist i. d. R. keine längeren Zykluszeiten auf und muss nach bestimmten Zeitabständen zwecks Zwischenreinigung abgefahren werden. Dies ist für den vorliegenden Fall der Aufarbeitung von Isocyanat-haltigen Destillationssumpfströmen aufgrund der erforderlichen Inertisierung des Reaktionsraumes und den hohen Temperaturen sowie der Anfahrproblematik nachteilig.

WO 2014/009342 A1 befasst sich mit einem Sprühtrocknungsverfahren zur Gewinnung von monomerem Isocyanat (d. h. das als Zielprodukt angestrebte, herzustellende Isocyanat im Unterschied zu unerwünschten hochmolekularen Isocyanatgruppen-haltigen Polymeren) aus Destillationsrückstand enthaltenden Sumpfströmen. Durch die beschriebene Sprühtrocknung werden ein getrockneter, von monomerem Isocyanat weitgehend bis vollständig befreiter Rückstand und ein monomeres Isocyanat umfassender Strom erhalten. Zur Durchführung dieses Verfahrens bedarf es eines speziellen Reaktors, sodass es im Allgemeinen nicht ohne größere Umbauten in eine vorhandene Isocyanat-Produktionsanlage integriert werden kann.

Weitere Verbesserungen auf dem Gebiet der Aufarbeitung von Isocyanat-Destillationssumpfströmen enthaltend neben Destillationsrückständen auch Anteile des herzustellenden Isocyanats sind daher wünschenswert. Insbesondere wäre es wünschenswert, das angestrebte Zielprodukt, d. i. das herzustellende Isocyanat, aus dem Destillationssumpfstrom auf einfache, betriebsstabile und ökonomische Weise abzutrennen, um Ausbeuteverluste und Wartungsaufwand auch im großtechnischen kontinuierlichen Betrieb möglichst gering zu halten, und zwar speziell auch dann, wenn die Zusammensetzung des den Rückstand enthaltenden Destillationssumpfstromes produktionsbedingten Schwankungen unterliegt. Wünschenswert wäre auch, wenn ein solches verbessertes Verfahren auf einfache Weise in ein bereits existierendes Verfahren, welches Trocknungsapparaturen des Standes der Technik (z. B. die erwähnten beheizten, produktumschaufelnden Vakuumtrockner mit horizontaler Welle) einsetzt, integriert werden könnte.

Dem vorstehend Gesagten Rechnung tragend betrifft die vorliegende Erfindung die **Aufarbeitung eines** aus

- dem herzustellenden Isocyanat,
- gegebenenfalls Leichtsiedern (insbesondere Lösungsmittel) und
- Destillationsrückstand

bestehenden **Destillationssumpfstroms,**
wobei dieser Destillationssumpfstrom aus der Aufarbeitung eines durch Phosgenierung des zu dem herzustellenden Isocyanat korrespondierenden primären Amins erhaltenen, das herzustellende Isocyanat umfassenden, flüssigen rohen Verfahrensprodukts, stammt, wobei die Aufarbeitung *des Destillationssumpfstroms* folgende Schritte umfasst:

1) optionale Vorkonzentrierung des Destillationssumpfstroms in einem Verdampfer durch partielle Verdampfung des in dem Destillationssumpfstrom enthaltenen herzustellenden Isocyanats, wobei ein an herzustellendem Isocyanat abgereicherter vorkonzentrierter flüssiger Strom erhalten wird;

2) Trocknung des Destillationssumpfstroms oder des in Schritt 1) erhaltenen vorkonzentrierten, an herzustellendem Isocyanat abgereicherten, flüssigen Stroms in einer Trocknungsvorrichtung bei einer Temperatur im Bereich von 150 °C bis 500 °C, bevorzugt im Bereich von 185 °C bis 300 °C, besonders bevorzugt im Bereich von 200 °C bis 270 °C, wobei herzustellendes Isocyanat unter Bildung eines festen Verfahrensprodukts verdampft und zurückgewonnen wird, wobei der auf die Gesamtmasse des der Trocknungsvorrichtung zugeführten Destillationsrückstandes bezogene Massenanteil an Carbodiimidgruppen-haltigen Verbindungen auf einen Wert von mindestens 15 %, bevorzugt mindestens 20 %, besonders bevorzugt mindestens 30 % eingestellt wird.

Vollkommen überraschend hat es sich herausgestellt, dass durch die erfindungsgemäße Vorgehensweise der Einstellung eines Mindestgehalts an Carbodiimidgruppen-haltigen Verbindungen im Trocknungsschritt 2) eine mit dem Durchtrocknen des Destillationsrückstands - infolge des unvermeidbaren Anstiegs der Viskosität - einhergehende gesteigerte mechanische Belastung der Trocknungsvorrichtung (z. B. Drehmomentanstieg bei Trocknungsvorrichtungen

mit einer Welle) vermindert oder sogar verhindert werden kann, was sich positiv auf die Betriebsstabilität der Trocknungsvorrichtung und damit auf die Betriebsstabilität des Gesamtverfahrens auswirkt.

Insbesondere ist ein Gegenstand der vorliegenden Erfindung ein **Verfahren zur Herstellung eines Isocyanats,** das die folgenden Schritte umfasst:

a) Phosgenierung des zu dem herzustellenden Isocyanat korrespondierenden primären Amins unter Erhalt eines das herzustellende Isocyanat umfassenden flüssigen rohen Verfahrensprodukts und eines Chlorwasserstoff umfassenden gasförmigen rohen Verfahrensprodukts, wobei optional ein Lösungsmittel eingesetzt wird;

b) Aufarbeitung des in Schritt a) erhaltenen flüssigen rohen Verfahrensprodukts umfassend die Schritte:

b.1) optionale Abtrennung von gelöstem Phosgen und gelöstem Chlorwasserstoff aus dem in Schritt a) erhaltenen flüssigen rohen Verfahrensprodukt unter Erhalt eines an Phosgen und Chlorwasserstoff abgereicherten flüssigen Verfahrensprodukts;

b.2) optionale Abtrennung von Lösungsmittel aus dem in Schritt a) erhaltenen flüssigen rohen Verfahrensprodukt oder aus dem in Schritt b) erhaltenen, an Phosgen und Chlorwasserstoff abgereicherten flüssigen Verfahrensprodukt unter Erhalt eines an Phosgen, Chlorwasserstoff und Lösungsmittel abgereicherten flüssigen Verfahrensprodukts;

b.3) Destillation des in Schritt a) erhaltenen flüssigen rohen Verfahrensprodukts oder des in Schritt b.1) erhaltenen, an Phosgen und Chlorwasserstoff abgereicherten flüssigen Verfahrensprodukts oder des in Schritt b.2) erhaltenen, an Phosgen, Chlorwasserstoff und Lösungsmittel abgereicherten flüssigen Verfahrensprodukts unter Erhalt eines einen ersten Teil des herzustellenden Isocyanats umfassenden Destillatstroms und eines aus Destillationsrückstand, einem zweiten Teil des herzustellenden Isocyanats und gegebenenfalls Leichtsiedern, insbesondere Lösungsmittel, bestehenden Destillationssumpfstroms;

c) Aufarbeitung des in Schritt b.3) erhaltenen Destillationssumpfstroms umfassend die Schritte:

c.1) optionale Vorkonzentrierung des in Schritt b) erhaltenen Destillationssumpfstroms in einem Verdampfer durch partielle Verdampfung des in dem in Schritt b) erhaltenen Destillationssumpfstrom enthaltenen zweiten Teil des herzustellenden Isocyanats, wobei ein an herzustellendem Isocyanat abgereicherter vorkonzentrierter flüssiger Strom erhalten wird;

c.2) Trocknung des in Schritt b) erhaltenen Destillationssumpfstroms oder des in Schritt c.1) erhaltenen vorkonzentrierten, an herzustellendem Isocyanat abgereicherten, flüssigen Stroms in einer Trocknungsvorrichtung bei einer Temperatur im Bereich von 150 °C bis 500 °C, bevorzugt im Bereich von 185 °C bis 300 °C, besonders bevorzugt im Bereich von 200 °C bis 270 °C, wobei herzustellendes Isocyanat unter Bildung eines festen Verfahrensprodukts verdampft und zurückgewonnen wird, wobei der auf die Gesamtmasse des der Trocknungsvorrichtung zugeführten Destillationsrückstandes bezogene Massenanteil an Carbodiimidgruppen-haltigen Verbindungen auf einen Wert von mindestens 15 %, bevorzugt mindestens 20 %, besonders bevorzugt mindestens 30 % eingestellt wird.

Dabei entspricht Schritt c) des **Verfahrens zur Herstellung eines Isocyanats** der zuvor beschriebenen **Aufarbeitung eines Destillationssumpfstroms,** und die Schritte c.1) und c.2) des Verfahrens zur Herstellung eines Isocyanats entsprechen den Schritten 1) und 2) der genannten Aufarbeitung eines Destillationssumpfstroms. Weiterhin entspricht Schritt b) des Verfahrens zur Herstellung eines Isocyanats der Aufarbeitung des durch Phosgenierung des zu dem herzustellenden Isocyanat korrespondierenden primären Amins erhaltenen, das herzustellende Isocyanat umfassenden, flüssigen rohen Verfahrensprodukts, aus welcher der Destillationssumpffstrom stammt.

Die Erfindung wird im Folgenden insbesondere anhand des Gesamtverfahrens umfassend die Schritte a) bis c) erläutert. Dies geschieht der Einfachheit halber und ist nicht so zu verstehen, dass diese Erläuterungen zwangsläufig auf das Gesamtverfahren beschränkt wären.

Als *"zu dem herzustellenden Isocyanat korrespondierenden primären Amin"* wird im Rahmen der vorliegenden Erfindung dasjenige Amin aufgefasst, das bei Umwandlung sämtlicher vorhandener $NH_2$-Gruppen in NCO-Gruppen das herzustellende Isocyanat ergibt.

Der *"Destillationssumpfstrom*" besteht erfindungsgemäß aus mindestens dem herzustellenden Isocyanat und dem im Folgenden definierten Destillationsrückstand. Daneben kann der Destillationssumpfstrom noch die im Folgenden definierten Leichtsieder enthalten. Der Destillationssumpfstrom besteht demnach erfindungsgemäß aus zwei (herzustellendes Isocyanat und Destillationsrückstand) oder drei (herzustellendes Isocyanat, Leichtsieder und Destillationsrückstand) Bestandteilen.

Unter *"Destillationsrückstand"* wird im Rahmen der vorliegenden Erfindung derjenige Anteil des in der Destillation aus Schritt b.3) erhaltenen Destillationssumpfstroms verstanden, der weder dem herzustellenden Isocyanat noch - sofern überhaupt vorhanden - Leichtsiedern zuzuordnen ist, wobei unter *"Leichtsiedern"* alle Stoffe oder azeotrop siedende Gemische von Stoffen verstanden werden, deren Siedepunkt geringer ist als der des herzustellenden Isocyanats, oder, sofern dieses Isocyanat als Isomerengemisch vorliegt, geringer ist als der des am niedrigsten siedenden Isomers des herzustellenden Isocyanats (im Fall von TDI also 2,6-TDI). Leichtsieder in diesem Sinne sind insbesondere gegebenenfalls eingesetztes Lösungsmittel aus Schritt a). Leichtsieder im Sinne von leicht siedenden Nebenkomponenten sind im Destillationssumpfstrom aus Schritt b.3) im Allgemeinen entweder gar nicht oder allenfalls in Spurenanteilen von bis zu 0,10 Massen-%, bevorzugt bis zu 0,010 Massen-%, besonders bevorzugt 0,0010 Massen-%, bezogen auf die Gesamtmasse des in Schritt b.3) erhaltenen Destillationssumpfstromes, vorhanden. Der Destillationsrückstand enthält solche Verbindungen, die unter den für Schritt b.3) gewählten Bedingungen von Druck und Temperatur nicht verdampfen oder die sich überhaupt nicht unzersetzt verdampfen lassen. Damit der Destillationssumpfstrom im Hinblick auf seine Fließeigenschaften gut bearbeitbar bleibt (d. h. nicht zu viskos oder gar fest wird), wird das herzustellende Isocyanat in Schritt b.3) nicht vollständig abdestilliert, sodass ein Teil desselben im Destillationssumpfstrom verbleibt. Es ist ebenfalls möglich, gegebenenfalls noch vorhandenes Lösungsmittel (aus Schritt a)) in Schritt b.3) nicht vollständig abzudestillieren und einen Restgehalt von Lösungsmittel im Destillationssumpfstrom von bis zu 10 Massen-%, bevorzugt von bis zu 1,0 Massen-%, bezogen auf die Gesamtmasse des in Schritt b.3) erhaltenen Destillationssumpfstromes, zuzulassen. Die schwer oder gar nicht verdampfbaren Verbindungen im Destillationsrückstand sind - sofern es sich nicht um den Phosgenierungsprozess unverändert durchlaufende Verunreinigungen aus dem eingesetzten primären Amin handelt - höhermolekulare Phosgenierungsprodukte, deren Struktur nicht immer exakt bekannt ist. So kann es sich um Verbindungen handeln, die sich (formal) von Polymerisationsprodukten des eingesetzten Amins durch Ersatz der nicht polymerisierten Amindurch Isocyanat-Gruppen ableiten lassen. Höhermolekulare Phosgenierungsprodukte können sich zum Teil (durch Weiterreaktion) auch noch in Schritt b) bilden.

Als höhermolekulare Phosgenierungsprodukte in diesem Sinne werden erfindungsgemäß auch *"Carbodiimidgruppen-haltige Verbindungen" aufgefasst, welche sich ebenfalls im Destillationssumpfstrom anreichern und* in der Terminologie der vorliegenden Erfindung als Bestandteil des Destillationsrückstands aufzufassen sind. Wie dem Fachmann bekannt ist, handelt es sich bei Carbodiimiden um eine Stoffgruppe organischer Verbindungen, die durch das Strukturmerkmal **R-N=C=N-R'** gekennzeichnet ist, wobei R und R' organische Reste bezeichnen. Bei der Phosgenierung primärer Amine können solche Verbindungen aus dem herzustellenden Isocyanat durch Abspaltung von Kohlenstoffdioxid entstehen. Im Fall eines Diamins entsteht das einfachste denkbare Carbodiimid durch die Ausbildung einer "-N=C=N-"-Brücke zwischen zwei Diisocyanat-Molekülen. In diesem Fall enthalten dann die beiden Reste R und R' ihrerseits noch freie NCO-Funktionen. Wenn diese mit herzustellendem Isocyanat oder weiteren Carbodiimiden mit freien NCO-Funktionen unter $CO_2$-Abspaltung weiterreagieren, kommt es zur Ausbildung von Strukturen mit mehreren "-N=C=N-"-Brücken und hohen Molmassen.

Der" *auf die Gesamtmasse des der Trocknungsvorrichtung zugeführten Destillationsrückstandes bezogene Massenanteil an Carbodiimidgruppen-haltigen Verbindungen* " in Schritt c.2) setzt sich additiv aus bis zu drei Beiträgen zusammen, nämlich aus

**(i)** der Masse an im Ausgangsmaterial von Schritt c.2) (also dem in Schritt b) erhaltenen Destillationssumpfstrom oder dem in Schritt c.1) erhaltenen vorkonzentrierten flüssigen Strom) vorhandenen Carbodiimidgruppen-haltigen Verbindungen,
**(ii)** der Masse an gegebenenfalls zugesetzten Carbodiimidgruppen-haltigen Verbindungen und
**(iii)** der Masse an in Schritt c.2) gegebenenfalls *in situ* gebildeten Carbodiimidgruppen-haltigen Verbindungen.

Erfindungsgemäß ist dafür Sorge zu tragen, dass die Summe aus **(i), (ii)** und **(iii),** dividiert durch die Gesamtmasse des Destillationsrückstandes, der der Trocknungsvorrichtung aus Schritt c.2) zugeführt wird, mindestens 0,15, bevorzugt mindestens 0,20, besonders bevorzugt mindestens 0,30 (entsprechend mindestens 15 Massen-%, bevorzugt mindestens 20 Massen-%, besonders bevorzugt mindestens 30 Massen-%) beträgt. Bei kontinuierlicher Verfahrensführung werden die entsprechenden *stündlichen Massenströme* zugrunde gelegt.

Es ist ausreichend, wenn lediglich einer der drei Anteile **(i)** bis **(iii)** von null verschieden und so groß ist, dass die erfindungsgemäßen Anforderungen erfüllt sind. Es ist beispielsweise möglich, den auf (i) und (iii) zurückzuführenden Anteil an Carbodiimidgruppen-haltigen Verbindungen durch geeignete Wahl der Prozessbindungen (siehe dazu Näheres weiter unten) auf null (oder nahezu null) zu halten und die erfindungsgemäßen Anforderungen allein durch Zugabe von Carbodiimidgruppen-haltigen Verbindungen aus einer externen Quelle zu erfüllen. Es ist aber auch möglich, auf eine solche externe Zugabe gemäß **(ii)** zu verzichten und die Einhaltung der erfindungsgemäßen Anforderungen an den Massenanteil an Carbodiimidgruppen-haltigen Verbindungen allein durch den Anteil gemäß **(i)** und/oder **(iii)** zu erfüllen.

Grundsätzlich können demnach drei Fälle unterschieden werden, die weiter unten noch detaillierter erläutert werden:

A. Einstellung von die Bildung von Carbodiimidgruppen-haltigen Verbindungen begünstigenden Verfahrensbedingungen in Schritt a) und/oder in Schritt b) und/oder - sofern durchgeführt - in Schritt c.1);

B. Herstellung von Carbodiimidgruppen-haltigen Verbindungen aus dem herzustellenden Isocyanat in einem separaten Prozess (z. B. durch thermische oder katalytisch induzierte Carbodiimidisierung des herzustellenden Isocyanats) und Zuführen der so hergestellten Carbodiimidgruppen-haltigen Verbindungen in die Trocknungsvorrichtung aus Schritt c.2);

C. *In* situ-Bildung (z. B. durch Tempern vor der eigentlichen Trocknung oder Durchführung der Trocknung bei relativ hohem Druck) von Carbodiimidgruppen-haltigen Verbindungen in Schritt c.2).

Die Fälle A, B und C können dabei sowohl einzeln als auch in Verbindung miteinander auftreten.

Es ist möglich, im Fall A die Verfahrensbedingungen in Schritt a) und/oder in Schritt b) und/oder - sofern durchgeführt - in Schritt c.1) so zu wählen, dass die über das Ausgangsmaterial von Schritt c.2) in diesen Schritt eingetragene Masse an Carbodiimidgruppen-haltigen Verbindungen so groß ist, dass die erfindungsgemäßen Anforderungen an den minimalen Massenanteil an Carbodiimidgruppen-haltigen im Destillationsrückstand erfüllt werden. In diesem Fall ist dann eine externe Herstellung oder eine In situ-Herstellung von Carbodiimidgruppen-haltigen Verbindungen nicht erforderlich.

Es ist ebenfalls möglich, im Fall B Carbodiimidgruppen-haltige Verbindungen in einem separaten Prozess aus dem herzustellen Isocyanat in einer solchen Menge herzustellen und dem Schritt c.2) zuzuführen, dass die erfindungsgemäßen Anforderungen an den minimalen Massenanteil an Carbodiimidgruppen-haltigen im Destillationsrückstand erfüllt werden. In diesem Fall ist dann die Einstellung von Verfahrensbedingungen gemäß Fall A oder eine In situ-Herstellung von Carbodiimidgruppen-haltigen Verbindungen nicht nötig.

Ebenso ist es möglich, im Fall C die komplette zur Erfüllung der erfindungsgemäßen Anforderungen an den minimalen Massenanteil an Carbodiimidgruppen-haltigen Verbindungen im Destillationsrückstand erforderliche Menge an Carbodiimidgruppen-haltigen Verbindungen in Schritt c.2) *in situ* zu bilden. In diesem Fall ist dann die Einstellung von Verfahrensbedingungen gemäß Fall A oder eine externe Herstellung von Carbodiimidgruppen-haltigen Verbindungen nicht nötig.

Es ist jedoch auch möglich, zur Einstellung des erfindungsgemäß geforderten, auf die Gesamtmasse des Destillationsrückstandes bezogenen Massenanteil an Carbodiimidgruppen-haltigen Verbindungen durch eine Kombination von zwei oder drei der genannten Fälle A, B und C zu bewirken.

Geeignete Verfahrensführungen für **Fall A** werden weiter unten noch im Detail erläutert. Zur Überprüfung, ob die für Schritt a) und/oder für Schritt b) und/oder - sofern durchgeführt - für Schritt c.1) gewählten Bedingungen geeignet sind, ist eine Bestimmung des Massenanteils der Carbodiimidgruppen-haltigen Verbindungen im zu trocknenden Ausgangsmaterial von Schritt c.2) erforderlich. Zu diesem Zweck sind dem Fachmann verschiedene analytische Methoden geläufig, die grundsätzlich alle angewandt werden können. Maßgeblich zur Beurteilung, ob die erfindungsgemäßen Anforderungen an den minimalen Massenanteil an Carbodiimidgruppen-haltigen Verbindungen im Destillationsrückstand erfüllt sind, ist jedoch die im Folgenden geschilderte Vorgehensweise (*"Messmethoden I und II"*):
Zunächst wird der Massenanteil an Carbodiimidgruppen-haltigen Verbindungen in dem zu trocknenden Ausgangsmaterial, das Schritt c.2) zugeführt wird, bestimmt. Dies geschieht mittels IR-Spektroskopie, wie im Folgenden geschildert (*"Messmethode I"*).

**<u>Messmethode I:</u>**

Eine auf Umgebungstemperatur abgekühlte Probe des zu untersuchenden Ausgangsmaterials von Schritt c.2) wird in ortho-Dichlorbenzol (fortan ODB) aufgelöst (der Massenanteil der Probe des Ausgangsmaterials von Schritt c.2), bezogen auf die Gesamtmasse der herzustellenden Lösung in ODB, wird dabei auf 20 % eingestellt) und im Verhältnis 1 : 1 geteilt. Ein Teil der aufgelösten Probe wird 15 min bei Umgebungsdruck unter Rückfluss gekocht (Heiztemperatur 190 °C) und anschließend in einem Wasser-Eis-Bad indirekt abgekühlt. Durch das Kochen wird Uretonimin, als welches Carbodiimid bei Raumtemperatur vorliegt, zu Carbodiimid und Isocyanat zurückgespalten. Der andere Teil der aufgelösten Probe bleibt unbehandelt. Die getemperte Probe wird in eine Küvette (Schichtdicke der Probe in der Küvette 150 bis 200 μm) gefüllt und in den Messstrahlengang eines IR-Spektrometers gesetzt. Die nicht getemperte Lösung wird in eine Küvette mit gleicher Schichtdicke wie die Messküvette gefüllt und in den Vergleichsstrahlengang des IR-Spektrometers eingesetzt. Von dem Bereich von 2600 $cm^{-1}$ bis 1900 $cm^{-1}$ wird ein IR-Spektrum aufgenommen. Die Extinktion E bei 2140 $cm^{-1}$ (Höhe des Signals) wird in Bezug auf die Basislinie im Bereich von 2500 $cm^{-1}$ bis 1900 $cm^{-1}$ bestimmt.

Der Uretonimingehalt ω(UI) in Massen-% (berechnet für die molare Masse M(UI) [in g/mol] des Uretonimins, das aus der Addition eines Mols des herzustellenden Isocyanats an ein Mol des durch Carbodiimidisierung zweier Mole des herzustellenden Isocyanats entstandenen Carbodiimids gebildet wird) wird nach folgender Gleichung berechnet:

$$\omega(UI) = [E(2140 cm^{-1}) \cdot M(UI) \cdot 100\ \%] / [d \cdot \varepsilon \cdot a].$$

Dabei bedeuten: M(UI) = molare Masse des Uretonimins wie zuvor definiert in *g/mol* (im Fall von TDI als dem herzustellenden Isocyanat 478); d = Schichtdicke der Probe in der Küvette in *cm*; ε = Extinktionskoeffizient in *l/mol · cm*; a = Konzentration der vermessenen Lösung in *g/l.*

Da 1 mol des Uretonimins aus 1 mol des Carbodiimids und einem mol des herzustellenden Isocyanats gebildet wird, kann der so ermittelte Massenanteil an Uretonimin leicht in den Massenanteil des Carbodimids ω(CD) umgerechnet werden:

$$\omega(CD) = \omega(UI) \cdot M(CD)/M(UI);$$

wobei M(CD) für die molare Masse in g/mol des Carbodiimids aus 2 mol des herzustellenden Isocyanats entspricht (im Fall von TDI als dem herzustellenden Isocyanat 304).

Der so ermittelte Wert für ω(CD) entspricht dem Massenanteil an Carbodiimidgruppen-haltigen Verbindungen in dem Schritt c.2) zugeführten *Ausgangsmaterial.* Die Bezugsgröße für die Quantifizierung des Massenanteils an Carbodiimidgruppen-haltigen Verbindungen ist erfindungsgemäß die Gesamtmasse *des der Trocknungsvorrichtung aus Schritt c.2) zugeführten Destillationsrückstandes.* Zur Bestimmung des so definierten Massenanteils an Carbodiimidgruppen-haltigen Verbindungen ist eine Quantifizierung des Massenanteils des Destillationsrückstandes in dem Schritt c.2) zugeführten Ausgangsmaterial erforderlich. Hierzu wird wie folgt vorgegangen (*"Messmethode II"*):

**Messmethode II:**

Eine ausgewogene Menge einer Probe des Schritt c.2) zuzuführenden Ausgangsmaterials wird in einer Labor-Destillationsapparatur, die mit einem Tropfenfänger (Destillationsaufsatz nach Reitmayer) ausgestattet ist, vorgelegt und bei einem Druck von 1,0 mbar und bei einer Heiztemperatur von 250 °C destilliert. Nach 30 Minuten (gemessen ab dem Zeitpunkt, an dem Druck und Temperatur die zuvor genannten Soll-Werte erreicht haben) wird die Heizung ausgeschaltet und die Apparatur abgekühlt. Das erhaltene Destillat und der nicht abdestillierte, im Vorlagekolben der Destillationsapparatur verbliebene Anteil der Probe werden gewogen. Der nicht destillierte Anteil der Probe wird in der Terminologie der vorliegenden Erfindung *als Destillationsrückstand im Sinne der Erfindung aufgefasst.* Durch Vergleich mit der bekannten Masse der in der Destillationsapparatur vorgelegten Probe wird der Massenanteil an Destillationsrückstand in dem Schritt c.2) zuzuführenden Ausgangsmaterial quantitativ bestimmt.

Mit diesem Ergebnis und dem Ergebnis für ω(CD) aus Messmethode I lässt sich der auf die Gesamtmasse des Destillationsrückstandes bezogene Massenanteil an Carbodiimidgruppen-haltigen Verbindungen leicht errechnen.

Sind geeignete Bedingungen für Schritt a) und/oder für Schritt b) und/oder - sofern durchgeführt - für Schritt c.1) auf diese Weise einmal festgelegt, kann auf eine Analyse des Ausgangsmaterials von Schritt c.2) im kontinuierlichen Betrieb grundsätzlich verzichtet werden. Es ist jedoch ratsam, von Zeit zu Zeit Kontrolluntersuchungen zur Überprüfung des einmal ermittelten Ergebnisses durchzuführen, insbesondere bei kontinuierlicher Fahrweise mindestens 1 x alle 720 h, bevorzugt mindestens 1 x alle 360 h, besonders bevorzugt mindestens 1 x alle 180 h, ganz besonders bevorzugt mindestens 1 x alle 90 h.

Wenn Fall A vorliegt und die Verfahrensbedingungen in Schritt a) und/oder in Schritt b) und/oder - sofern durchgeführt - in Schritt c.1) so gewählt sind, dass die über das Ausgangsmaterial von Schritt c.2) in diesen Schritt eingetragene Masse an Carbodiimidgruppen-haltigen Verbindungen bereits hinreichend groß ist, um die erfindungsgemäßen Anforderungen an den minimalen Massenanteil an Carbodiimidgruppen-haltigen im Destillationsrückstand zu erfüllen, kann auf eine Messung des auf **(iii)** zurückgehenden Anteils an Carbodiimidgruppen-haltigen Verbindungen verzichtet werden, da es keine Rolle spielt, ob in Schritt c.2) *in situ* weitere Carbodiimidgruppen-haltige Verbindungen gebildet werden oder nicht (eine *Verringerung* des Anteils an Carbodiimidgruppen-haltigen Verbindungen in Schritt c.2) ist chemisch nicht zu erwarten). Selbstverständlich kann in diesem Fall auch eine Zugabe von Carbodiimidgruppen-haltigen Verbindungen gemäß **(ii)** unterbleiben.

Liegt dieser Fall nicht vor, so ist erfindungsgemäß auf andere Weise dafür Sorge zu tragen, dass Carbodiimidgruppen-haltige Verbindungen in ausreichender Menge in Schritt c.2) vorhanden sind.

Dies kann im **Fall B** durch Zugabe von Carbodiimidgruppen-haltigen Verbindungen aus einer externen Quelle geschehen. *Der maßgebliche Massenanteil an Carbodiimidgruppen-haltigen Verbindungen in dem zugegebenen Ma-*

*terial aus externer Quelle ist der gemäß Messemethode I bestimmte.* Je nach Menge des zugegebenen Materials und dessen Massenanteil an Carbodiimidgruppen-haltigen Verbindungen ist auch ohne Quantifizierung des Destillationsrückstandes klar, ob die erfindungsgemäßen Anforderungen erfüllt werden (nämlich dann, wenn selbst unter der Annahme, dass der Destillationssumpf vollständig aus Rückstand besteht, der auf die Gesamtmasse des Destillationsrückstands bezogene erfindungsgemäß geforderte Mindestmassenanteil an Carbodiimidgruppen-haltigen Verbindungen erreicht ist). Sollte eine Quantifizierung des Destillationsrückstandes nötig sein, *so ist die Bestimmung gemäß Messmethode II maßgeblich.*

Ebenso ist es möglich, durch die Wahl geeigneter Verfahrensbedingungen dafür Sorge zu tragen, dass Carbodiimidgruppen-haltige Verbindungen in Schritt c.2) *in situ* gebildet werden. Geeignete Verfahrensführungen für diesen **Fall C** werden weiter unten noch im Detail erläutert. Zur Überprüfung, ob die für Schritt c.2) gewählten Bedingungen hierfür geeignet sind, wird die Massenbilanz der Trocknung aus Schritt c.2) wie folgt kontrolliert (*"Messmethode III"*):

**Messmethode III:**

Der Trocknungsvorrichtung aus Schritt c.2) wird ein zu trocknendes Ausgangsmaterial mit einem bekannten (mit Messmethode II bestimmten) Massenanteil an Destillationsrückstand zugeführt. Das in der beschriebenen Destillation gemäß Messmethode II erhaltene Destillat besteht aus dem herzustellenden Isocyanat und gegebenenfalls Leichtsiedern (insbesondere aus Schritt a) stammendes Lösungsmittel). Die Zusammensetzung des Destillats (Verhältnis Leichtsieder zu herzustellendem Isocyanat) wird gaschromatographisch quantifiziert (HP-5, 30m*320μm*0,25μm, 40-250°C, 1-20°C/min, TCD-Detektor).

Säule: Agilent 19091J-413: 1300.52926, HP-5, 5% Phenyl Methyl Siloxan, 325 °C: 30 m x 320 μm x 0.25 μm.

Heizrampe: 40 °C Starttemperatur, dann 1 °C/min bis 45 °C, dann 20 °C/min bis 250 °C, 250 °C für 5 min halten.

Laufzeit: 20,25 min.

Detektor: TCD (Wärmeleitfähigkeitsdetektor).

Auf diese Weise wird ermittelt, wie viel herzustellendes Isocyanat aus dem in Schritt c.2) zu trocknendem Ausgangsmaterial abdestilliert werden kann. In Schritt c.2) wird herzustellendes Isocyanat unter Bildung eines festen Verfahrensproduktes verdampft und zurückgewonnen. Die Masse (bei kontinuierlicher Verfahrensführung der *stündliche Massenstrom*) des zurückgewonnenen Teils des herzustellenden Isocyanats wird ermittelt und mit der wie zuvor beschrieben ermittelten Masse (bei kontinuierlicher Verfahrensführung mit dem *stündlichen Massenstrom*) des über das Ausgangsmaterial zugeführten herzustellenden Isocyanats verglichen. *Die ermittelte Massendifferenz (bei kontinuierlicher Verfahrensführung die Differenz im stündlichen Massenstrom) wird erfindungsgemäß als auf die Bildung Carbodiimidgruppen-haltiger Verbindungen zurückzuführen aufgefasst,* d. h. *diese Massendifferenz dividiert durch die Masse des der Trocknungsvorrichtung aus Schritt c.2) zugeführten Destillationsrückstandes ergibt den erfindungsgemäß wesentlichen Wert des auf die Gesamtmasse des der Trocknungsvorrichtung zugeführten Destillationsrückstandes bezogenen Massenanteils an Carbodiimidgruppen-haltigen Verbindungen.*

Ist die Menge der *in situ* gebildeten Carbodiimidgruppen-haltigen Verbindungen groß genug, um die erfindungsgemäßen Anforderungen an den Minimalgehalt im Destillationsrückstand zu erfüllen, kann auf die für Fall A beschriebene analytische Bestimmung gemäß Messmethode I verzichtet werden. Sind geeignete Bedingungen für Schritt c.2) auf diese Weise einmal festgelegt, kann auf eine fortwährende Überprüfung des Gehalts an *in situ* gebildeten Carbodiimidgruppen-haltigen Verbindungen während des Betriebs der in Schritt c.2) eingesetzten Trocknungsvorrichtung grundsätzlich verzichtet werden. Es ist jedoch ratsam, von Zeit zu Zeit Kontrolluntersuchungen zur Überprüfung des einmal ermittelten Ergebnisses durchzuführen, insbesondere bei kontinuierlicher Fahrweise mindestens 1 x alle 720 h, bevorzugt mindestens 1 x alle 360 h, besonders bevorzugt mindestens 1 x alle 180 h, ganz besonders bevorzugt mindestens 1 x alle 90 h.

Es folgt zunächst eine Kurzzusammenfassung verschiedener möglicher **Ausführungsformen der Erfindung:** In einer **ersten Ausführungsform der Erfindung** wird die Phosgenierung in der Flüssigphase in Gegenwart eines Lösungsmittels durchgeführt.

In einer **zweiten Ausführungsform der Erfindung** wird die Phosgenierung in der Gasphase durchgeführt, wobei die Phosgenierung eine Quenche umfasst, in der das gebildete gasförmige Verfahrensprodukt enthaltend das herzustellende Isocyanat durch Inkontaktbringen mit einer Quenchflüssigkeit ausgewählt aus der Gruppe bestehend aus Lösungsmittel, dem herzustellenden Isocyanat und Gemischen aus dem herzustellenden Isocyanat und Lösungsmittel, abgekühlt und das herzustellende Isocyanat verflüssigt wird.

In einer **dritten Ausführungsform der Erfindung,** die eine besondere Ausgestaltung der zweiten Ausführungsform ist, wird die Quenchflüssigkeit ausgewählt aus der Gruppe bestehend aus Lösungsmittel und Gemischen aus dem herzustellenden Isocyanat und Lösungsmittel.

In einer **vierten Ausführungsform der Erfindung,** die eine besondere Ausgestaltung aller Ausführungsformen, in denen der Einsatz eines Lösungsmittels vorgesehen ist, ist, wird dieses Lösungsmittel ausgewählt aus der Gruppe bestehend aus Chlorbenzol, ortho-Dichlorbenzol, para-Dichlorbenzol, den Isomeren des Trichlorbenzols, Toluol,

den Isomeren des Xylols und Mischungen der vorgenannten Lösungsmittel.

In einer **fünften Ausführungsform der Erfindung,** die ebenfalls eine besondere Ausgestaltung aller Ausführungsformen, in denen der Einsatz eines Lösungsmittels vorgesehen ist, ist, bestehen die im Destillationssumpfstrom gegebenenfalls vorhandenen Leichtsieder aus Lösungsmittel und Nebenkomponenten, wobei der Massenanteil an Nebenkomponenten, bezogen auf die Gesamtmasse des Destillationssumpfstromes, maximal 0,10 Massen-% beträgt.

In einer **sechsten Ausführungsform der Erfindung,** die mit allen anderen Ausführungsformen kombiniert werden kann, ist der Schritt 1) umfasst, wobei die Vorkonzentrierung bei einer Temperatur im Bereich von 120 °C bis 180 °C und bei einem Druck im Bereich von 20 mbar$_{(abs.)}$ bis 60 mbar$_{(abs.)}$ erfolgt.

In einer **siebten Ausführungsform der Erfindung,** die mit allen anderen Ausführungsformen kombiniert werden kann, wird als Trocknungsvorrichtung in Schritt 2) ein Apparat ausgewählt aus der Gruppe bestehend aus beheizten, produktumschaufelnden Vakuumtrocknern mit horizontaler Welle, Drehrohren, Scheibentrocknern, Bandtrocknern und Granulierschnecken eingesetzt.

In einer **achten Ausführungsform der Erfindung,** die mit allen anderen Ausführungsformen kombiniert werden kann, umfasst das erfindungsgemäße Verfahren die folgenden Schritte:

a) Phosgenierung des zu dem herzustellenden Isocyanat korrespondierenden primären Amins unter Erhalt eines das herzustellende Isocyanat umfassenden flüssigen rohen Verfahrensprodukts und eines Chlorwasserstoff umfassenden gasförmigen rohen Verfahrensprodukts, wobei optional ein Lösungsmittel eingesetzt wird;

b) Aufarbeitung des in Schritt a) erhaltenen flüssigen rohen Verfahrensprodukts umfassend die Schritte:

b.1) optionale Abtrennung von gelöstem Phosgen und gelöstem Chlorwasserstoff aus dem in Schritt a) erhaltenen flüssigen rohen Verfahrensprodukt unter Erhalt eines an Phosgen und Chlorwasserstoff abgereicherten flüssigen Verfahrensprodukts;

b.2) optionale Abtrennung von Lösungsmittel aus dem in Schritt a) erhaltenen flüssigen rohen Verfahrensprodukt oder aus dem in Schritt b) erhaltenen, an Phosgen und Chlorwasserstoff abgereicherten flüssigen Verfahrensprodukt unter Erhalt eines an Phosgen, Chlorwasserstoff und Lösungsmittel abgereicherten flüssigen Verfahrensprodukts;

b.3) Destillation des in Schritt a) erhaltenen flüssigen rohen Verfahrensprodukts oder des in Schritt b.1) erhaltenen, an Phosgen und Chlorwasserstoff abgereicherten flüssigen Verfahrensprodukts oder des in Schritt b.2) erhaltenen, an Phosgen, Chlorwasserstoff und Lösungsmittel abgereicherten flüssigen Verfahrensprodukts unter Erhalt eines einen ersten Teil des herzustellenden Isocyanats umfassenden Destillatstroms und eines aus Destillationsrückstand, einem zweiten Teil des herzustellenden Isocyanats und gegebenenfalls Leichtsiedern, insbesondere Lösungsmittel bestehenden Destillationssumpfstroms;
wobei der in Schritt b.3) erhaltene Destillationssumpfstrom der in Schritt 2) oder in Schritt 1) und Schritt 2) aufzuarbeitende Destillationssumpfstrom ist.

In einer **neunten Ausführungsform der Erfindung,** die insbesondere aus besondere Ausgestaltung der achten Ausführungsform eingesetzt wird, wird die Einstellung des auf die Gesamtmasse des der Trocknungsvorrichtung zugeführten Destillationsrückstandes bezogenen Massenanteils an Carbodiimidgruppen-haltigen Verbindungen über ausschließlich eine, zwei oder alle der folgenden Maßnahmen bewirkt:

A. Einstellung von die Bildung von Carbodiimidgruppen-haltigen Verbindungen begünstigenden Verfahrensbedingungen in Schritt a) und/oder in Schritt b) und/oder - sofern durchgeführt - in Schritt 1);

B. Herstellung von Carbodiimidgruppen-haltigen Verbindungen aus dem herzustellen Isocyanat in einem separaten Prozess und Zuführen der so hergestellten Carbodiimidgruppen-haltigen Verbindungen in die Trocknungsvorrichtung aus Schritt 2);

C. *In situ*-Bildung von Carbodiimidgruppen-haltigen Verbindungen in Schritt 2).

In einer **zehnten Ausführungsform der Erfindung,** die eine besondere Ausgestaltung der neunten Ausführungsform ist, ist die Maßnahme A umfasst, wobei die Einstellung von die Bildung von Carbodiimidgruppen-haltigen Verbindungen begünstigenden Verfahrensbedingungen in Schritt a) und/oder in Schritt b) und/oder - sofern durchgeführt - in Schritt 1) durch eine Erhöhung der Temperatur im jeweiligen Schritt und/oder durch eine Erhöhung der Verweilzeit des den jeweiligen Schritt durchlaufenden Verfahrensprodukts bewirkt wird.

In einer **elften Ausführungsform der Erfindung,** die ebenfalls eine besondere Ausgestaltung der neunten Ausführungsform ist, aber auch mit der zehnten und/oder der weiter unten geschilderten vierzehnten Ausführungsform gemeinsam angewendet werden kann, ist die Maßnahme B umfasst, wobei die Herstellung von Carbodiimidgruppenhaltigen Verbindungen aus dem herzustellen Isocyanat in einem separaten Prozess Folgendes umfasst: Temperung eines das herzustellende Isocyanat enthaltenden Verfahrensprodukts aus dem erfindungsgemäßen Herstellverfahren

in Abwesenheit von Katalysatoren bei einer Temperatur im Bereich von 200 °C bis 270 °C
oder

in Gegenwart von Katalysatoren vom Phospholinoxid bei einer Temperatur im Bereich von 50 °C bis 150 °C.

In einer **zwölften Ausführungsform der Erfindung,** die eine besondere Ausgestaltung der zehnten und/oder elften Ausführungsform ist, und zwar insbesondere dann, wenn auf die Maßnahme C) verzichtet wird, wird Schritt 2) bei einem Druck im Bereich von 10 mbar$_{(abs.)}$ bis 250 mbar$_{(abs.)}$, bevorzugt im Bereich von 20 mbar$_{(abs.)}$ bis 200 mbar$_{(abs.)}$, besonders bevorzugt im Bereich von 30 mbar$_{(abs.)}$ bis 100 mbar$_{(abs.)}$ durchgeführt.

In einer **dreizehnten Ausführungsform der Erfindung,** die ebenfalls eine besondere Ausgestaltung der neunten Ausführungsform ist, aber auch mit der zehnten und/oder der elften Ausführungsform gemeinsam angewendet werden kann, ist die Maßnahme C umfasst, wobei die Trocknung gemäß Schritt 2)

zunächst in einem ersten Teilschritt 2.1) bei einem Druck im Bereich von > 750 mbar$_{(abs.)}$ bis 1013 mbar$_{(abs)}$ und bei einer Temperatur im Bereich von 200 °C bis 270 °C und dann in einem zweiten Teilschritt 2.2) bei einem Druck im Bereich von 10 mbar$_{(abs)}$ bis 250 mbar$_{(abs)}$, bevorzugt 20 mbar$_{(abs.)}$ bis 200 mbar$_{(abs.)}$, besonders bevorzugt 30 mbar$_{(abs.)}$ bis 100 mbar$_{(abs)}$, und bei einer Temperatur im Bereich 200 °C bis 270 °C durchgeführt wird.
oder

bei einem Druck im Bereich von > 250 mbar$_{(abs)}$ bis 750 mbar$_{(abs)}$, bevorzugt 300 mbar$_{(abs)}$ bis 650 mbar$_{(abs)}$, besonders bevorzugt 450 mbar$_{(abs)}$ bis 550 mbar$_{(abs)}$ und einer Temperatur im Bereich von 200 °C bis 270 °C

durchgeführt wird.

In einer **vierzehnten Ausführungsform der Erfindung,** die mit allen anderen Ausführungsformen kombiniert werden kann, wird das herzustellende Isocyanat ausgewählt aus der Gruppe bestehend aus Toluylendiisocyanat, Naphthyldiisocyanat, 1,5-Pentandiisocyanat, 1,6-Hexamethylendiisocyanat, Isophorondiisocyanat, Xylylendiisocyanat und Diisocyanatodicyclohexylmethan.

Die zuvor kurz geschilderten Ausführungsformen und weitere mögliche Ausgestaltungen der Erfindung werden im Folgenden näher erläutert. Dabei wird der Einfachheit halber auf das Gesamtverfahren mit den Schritten a) bis c) Bezug genommen (*"achte Ausführungsform der Erfindung*"), was nicht bedeutet, dass alle im Folgenden geschilderten möglichen Ausgestaltungen der Erfindung auf diese achte Ausführungsform beschränkt sind. Verschiedene Ausführungsformen sind, sofern sich aus dem Kontext für den Fachmann nicht eindeutig das Gegenteil ergibt, beliebig miteinander kombinierbar.

In **Schritt a)** wird zunächst das herzustellende Isocyanat durch Phosgenierung des korrespondierenden primären Amins in roher Form (d. h. als *"das herzustellende Isocyanat umfassende flüssige rohe Verfahrensprodukt"*) gewonnen. Das herzustellende Isocyanat ist dabei bevorzugt ein Diisocyanat, und zwar besonders bevorzugt ein Diisocyanat ausgewählt aus der Gruppe bestehend aus Toluylendiisocyanat (nachfolgend TDI), Naphthyldiisocyanat (nachfolgend NDI), 1,5-Pentandiisocyanat (nachfolgend PDI), 1,6-Hexamethylendiisocyanat (nachfolgend HDI), Isophorondiisocyanat (nachfolgend IDPI), Xylylendiisocyanat (nachfolgend XDI) und Diisocyanatodicyclohexylmethan (nachfolgend H12-MDI). Die zu den zuvor genannten Isocyanaten korrespondierenden primären Amine sind Toluylendiamin (nachfolgend TDA), Naphthylendiamin (nachfolgend NDA), 1,5-Pentandiamin (nachfolgend PDA), 1,6-Hexamethylendiamin (nachfolgend HDA), Isophorondiamin (nachfolgend IDPA), Xylylendiamin (nachfolgend XDA) und Diaminodicyclohexylmethan (nachfolgend H12-MDA). Sofern die genannten Amine in verschiedenen isomeren Formen vorliegen können, ohne dass diese explizit angegeben sind, sind erfindungsgemäß alle Isomerenverteilungen umfasst. Liegt das korrespondierende primäre Amin als Gemisch aus verschiedenen Isomeren vor, so entspricht die Isomerenverteilung des herzustellenden Isocyanats im Wesentlichen oder vollständig der des Ausgangsamins. Prinzipiell können auch Gemische der vorgenannten Amine umgesetzt werden, obwohl dies im Allgemeinen nicht bevorzugt ist.

Besonders bevorzugt wird das erfindungsgemäße Verfahren zur Herstellung von TDI durch Phosgenierung von TDA eingesetzt. Die genaue jeweils vorliegende Isomerenzusammensetzung des eingesetzten TDA ist für das erfindungsgemäße Verfahren nicht von Belang. Üblicherweise umfasst TDA, welches bevorzugt eingesetzt wird, 78,0

Massen-% bis 82,0 Massen-% 2,4-TDA und 18,0 Massen-% bis 22,0 Massen-% 2,6-TDA, bezogen auf die Gesamtmasse der 2,4- und 2,6-TDA-Isomeren. Bezogen auf die Gesamtmasse des TDA machen dabei die 2,4- und 2,6-TDA-Isomeren bevorzugt in Summe von 95,0 Massen-% bis 100 Massen-%, besonders bevorzugt von 98,0 Massen-% bis 100 Massen-%, aus.

Die Phosgenierung eines primären Amins zum korrespondierenden Isocyanat ist grundsätzlich bekannt und kann nach einem der im Stand der Technik bekannten Verfahren durchgeführt werden.

Als Beispiele seien die in den folgenden Literaturstellen beschriebenen Verfahren genannt: Ullmann's Encyclopedia of Industrial Chemistry, 5th ed. Vol. A 19, S. 390 ff., VCH Verlagsgesellschaft mbH, Weinheim, 1991, G. Oertel (Ed.) Polyurethane Handbook, 2nd Edition, Hanser Verlag, München, 1993, S. 60 ff., G. Wegener et. A1. Applied Catalysis A: General 221 (2001), S. 303 bis 335, Elsevier Science B.V., EP 1 369412 A1, EP 1 754698 B1 und EP 0 289 840 B1.

Bevorzugt findet die Umsetzung von primärem Amin und Phosgen in Schritt a) wie folgt statt:
Phosgen wird in einem stöchiometrischen Überschuss, bezogen auf das primäre Amin, eingesetzt. Die Phosgenierung kann in der Flüssig- und in der Gasphase, insbesondere in der Gasphase, durchgeführt werden.

Beispiele für **Flüssigphasenphosgenierungen** sind in DE 37 44 001 C1, EP 0 314 985 A1, EP 1369 412 A1, DE-A-102 60 027, DE-A-102 60 093, DE-A 103 10 888, DE-A-10 2006 022 448, US-A 2007/0299279 und der dort jeweils zitierten Literatur beschrieben.

In einer bevorzugten Ausgestaltung des Schritts a) als Flüssigphasenphosgenierung wird wie folgt verfahren: Die Reaktionspartner primäres Amin und Phosgen werden getrennt voneinander in einem Lösungsmittel gelöst. Bevorzugte Lösungsmittel für diesen Zweck sind Chlorbenzol, ortho-Dichlorbenzol, para-Dichlorbenzol, die Isomere des Trichlorbenzols, Toluol und/oder die Isomere des Xylols. Besonders bevorzugte Lösungsmittel sind Chlorbenzol und Dichlorbenzol, außerordentlich besonders bevorzugt ist ortho-Dichlorbenzol, und zwar insbesondere in Verbindung mit TDI als dem herzustellenden Isocyanat. Das primäre Amin wird bevorzugt in einer Konzentration von 10 Massen-% bis 40 Massen-%, bevorzugt von 10 Massen-% bis 20 Massen-%, bezogen auf die Gesamtmasse der Lösung, verwendet. Phosgen wird bevorzugt in einer Konzentration von 10 Massen-% bis 40 Massen-%, bevorzugt von 25 Massen-% bis 35 Massen-%, bezogen auf die Gesamtmasse der Lösung, verwendet.

Eine effiziente Vermischung von primärem Amin und Phosgen ist beim Flüssigphasenverfahren von hoher Bedeutung. Hierzu kommen im Stand der Technik statische (bevorzugt Düsen) und dynamische (enthaltend mechanisch bewegte Teile) Mischeinrichtungen zum Einsatz. Nach der Vermischung durchlaufen die vermischten Reaktionspartner eine Reaktionszone zur Umsatzvervollständigung. Mischeinrichtung und Reaktionszone können auch in einem gemeinsamen Reaktor angeordnet sein. Phosgen wird bevorzugt im stöchiometrischen Überschuss gegenüber den primären Aminogruppen des Amins eingesetzt, insbesondere in einem molaren Verhältnis von Phosgen zu primären Aminogruppen im Bereich von 4 : 1 bis 1 : 1, besonders bevorzugt im Bereich von 3 : 1 bis 1 : 1, ganz besonders bevorzugt im Bereich von 2 : 1 bis 1 : 1.

Die Flüssigphasenphosgenierung kann bei verschiedenen Temperatur- und Druckniveaus durchgeführt werden. So ist es beispielsweise möglich, die Flüssigphasenphosgenierung bei einer Temperatur im Bereich von 0 °C bis 240 °C, bevorzugt von 20 °C bis 240 °C und bei einem Druck im Bereich von 1,0 $bar_{(abs.)}$ bis 70 $bar_{(abs.)}$, bevorzugt von 1,0 $bar_{(abs.)}$ bis 50 $bar_{(abs.)}$, durchzuführen.

Der bei der Reaktion als Koppelprodukt entstehende Chlorwasserstoff liegt zum Teil gelöst in der flüssigen Phase vor und gast zum Teil aus. Wie groß der Anteil des gelösten gegenüber dem gasförmig vorliegenden Chlorwasserstoff ist, hängt vom gewählten Temperatur- und Druckniveau ab. In jedem Fall werden in Schritt a) ein flüssiger, das herzustellende Isocyanat und Lösungsmittel enthaltender Strom und ein gasförmiger, Chlorwasserstoff und gegebenenfalls verdampftes Lösungsmittel enthaltender Strom erhalten. Wenn Phosgen überstöchiometrisch eingesetzt wird, enthalten beide Ströme zudem Phosgen. Beide Ströme können direkt der Reaktionszone entnommen werden. Es ist auch möglich, der Reaktionszone ein zweiphasiges Verfahrensprodukt (enthaltend eine Flüssig- und eine Gasphase) zu entnehmen und dieses in eine Vorrichtung zur Phasentrennung zu überführen. Diese Phasentrennung kann allen dem Fachmann bekannten Apparaten, die zur Trennung von Gas- und Flüssigphasen geeignet sind, erfolgen. Bevorzugt werden Gas- und Flüssigkeitsabscheider wie z. B. Zyklonabscheider, Umlenkabscheider und/oder Schwerkraftabscheider mit und ohne statische Abscheidehilfe eingesetzt. Es ist ebenfalls möglich, die Phasentrennung durch Verminderung des Drucks gegenüber dem in der Reaktionszone herrschendem Druck infolge verstärkten Ausgasens von Chlorwasserstoff (und gegebenenfalls anderer gasförmiger Bestandteile) zu unterstützen. Der der Reaktionszone entnommene flüssige Strom bzw. - sofern vorhanden - der dem der Reaktionszone nachgeschalteten Apparat zur Phasentrennung entnommene flüssige Strom ist in dieser Ausführungsform das Ausgangsmaterial der in Schritt b) durchzuführenden Aufarbeitung, d. h. diese Flüssigphase ist in der Terminologie der vorliegenden Erfindung *"das herzustellende Isocyanat umfassende flüssige rohe Verfahrensprodukt*".

Am Beispiel des primären Amins TDA wird die Flüssigphasenphosgenierung im Folgenden noch näher geschildert:
Im Flüssigphasenverfahren wird das TDA, gegebenenfalls bereits in einem der weiter oben definierten inerten Lösungsmittel gelöst, der Vermischung mit Phosgen bei einer Temperatur im Bereich von -10 °C bis 220 °C, bevorzugt von 0°

C bis 200 °C, besonders bevorzugt von 20 °C bis 180 °C, zugeführt. Das Phosgen kann der Vermischung mit TDA entweder ohne Lösungsmittel oder ebenfalls in einem der weiter oben definierten Lösungsmittel gelöst mit einer Temperatur im Bereich von -40 °C bis 200 °C, bevorzugt von -30 °C bis 170 °C, besonders bevorzugt von -20 °C bis 150 °C zugeführt, werden. Die Vermischung von TDA und Phosgen, gegebenenfalls bereits in einem der weiter oben definierten inerten Lösungsmittel gelöst, erfolgt im Flüssigphasenverfahren bevorzugt mittels eines statischen Mischers oder eines dynamischen Mischers. Beispiele für geeignete statische Mischer sind u. a. Düsen bzw. Düsenanordnungen, wie z. B. in DE 17 92 660 A, US 4,289,732 oder US 4,419,295 beschrieben. Beispiele für geeignete dynamische Mischer sind u. a. pumpenähnliche Aggregate, wie beispielsweise Kreiselpumpen (vgl. US 3,713,833) oder spezielle Mischer-Reaktoren (vgl. EP 0 291 819 A, EP 0 291 820 A, EP 0 830 894 A).

Die Umsetzung in der sich anschließenden Reaktionszone erfolgt im Flüssigphasenverfahren bei einer Temperatur im Bereich von 0 °C bis 250 °C, bevorzugt von 20 °C bis 200 °C, besonders bevorzugt von 20 °C bis 180 °C, mit einer mittleren Verweilzeit des Reaktionsgemisches in der Reaktionszone im Bereich von 10 s bis 5 h, bevorzugt von 30 s bis 4 h, besonders bevorzugt von 60 s bis 3 h, und bei einem Druck von bis zu 100 $bar_{(abs.)}$, bevorzugt im Bereich von 1,0 $bar_{(abs.)}$ bis 70 bar($_{abs}$.), besonders bevorzugt von 1,0 $bar_{(abs.)}$ bis 50 $bar_{(abs)}$. Beispiele für erfindungsgemäß einsetzbare Verfahrensführungen im Hinblick auf die Umsetzung in der Reaktionszone sind z. B. in US-A 2007/0299279 (insbesondere S. 7, Abschnitte [0070], [0071], [0089]) und DE-A 103 10 888 (insbesondere S. 5 Abschnitte [0038], [0039]) und den darin jeweils zitierten Schriften beschrieben.

Beispiele für **Gasphasenphosgenierungen** sind in EP 0 570 799 A1, EP 1 555 258 A1, EP 1 526 129 A1 und DE 101 61 384 A1, sowie insbesondere für aliphatische Isocyanate in EP 0 289 840 B1, EP 1 754 698 B1, EP 1 319 655 B1 und EP 1 362 847 B1 beschrieben. Vorteile dieses Verfahrens gegenüber der ansonsten üblichen Flüssigphasenphosgenierung liegen in der Energieeinsparung, bedingt durch die Minimierung eines aufwändigen Lösungsmittel- und Phosgenkreislaufs.

In einer bevorzugten Ausgestaltung des Schritts a) als Gasphasenphosgenierung wird wie folgt verfahren: In einem Schritt a.1) wird ein gasförmiger Strom eines primären Amins bereitgestellt. Geeignete Methoden hierfür sind dem Fachmann grundsätzlich bekannt. Im Folgenden werden bevorzugte Ausführungsformen geschildert.

Die Überführung des primären Amins in die Gasphase kann in allen aus dem Stand der Technik bekannten Verdampfungsapparaturen erfolgen, insbesondere in einem Fallfilmverdampfer. Bevorzugt werden solche Verdampfungsapparaturen eingesetzt, bei denen ein kleiner Arbeitsinhalt mit einer hohen Umwälzleistung über einen Fallfilmverdampfer geführt wird.

Zur Minimierung der thermischen Belastung des Amins ist es unabhängig von der genauen Ausgestaltung der Verdampfungsapparatur bevorzugt, den Verdampfungsvorgang durch Zuspeisung eines Inertgases wie $N_2$, He, Ar (insbesondere $N_2$) oder der Dämpfe eines inerten Lösungsmittels, bevorzugt ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel, zu unterstützen.

Weiterhin erfolgt die Verdampfung - und erforderlichenfalls Überhitzung - des Ausgangsamins (insbesondere auf eine Temperatur im Bereich von 200 °C bis 430 °C, bevorzugt 250 °C bis 420 °C, besonders bevorzugt 250 °C bis 400 °C) bevorzugt mehrstufig, um nicht verdampfte Tröpfchen im gasförmigen Aminstrom zu vermeiden. Insbesondere bevorzugt sind mehrstufige Verdampfungs- und Überhitzungsschritte, in denen zwischen den Verdampfungs- und Überhitzungssystemen Tröpfchenabscheider eingebaut sind und / oder die Verdampfungsapparaturen auch die Funktion eines Tröpfchenabscheiders besitzen. Geeignete Tröpfchenabscheider sind dem Fachmann bekannt.

In einem Schritt a.2) wird ein gasförmiger Phosgenstrom bereitgestellt. Bevorzugt wird ein molares Verhältnis von Phosgen zu primären Amingruppen von 1,1 : 1 bis 20 : 1, besonders bevorzugt 1,2 : 1 bis 5,0 : 1 eingestellt. Auch das Phosgen wird bevorzugt, wie zuvor für das primäre Amin beschrieben, auf eine Temperatur im Bereich von 200 °C bis 430 °C, bevorzugt 250 °C bis 420 °C, besonders bevorzugt 250 °C bis 400 °C, erhitzt und gegebenenfalls verdünnt mit einem Inertgas wie $N_2$, He, Ar (insbesondere $N_2$) oder mit den Dämpfen eines inerten Lösungsmittels, bevorzugt ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel.

In einem Schritt a.3) werden die Reaktionspartner primäres Amin und Phosgen in einer Mischzone vermischt und in einer sich anschließenden Reaktionszone umgesetzt. Die getrennt aufgeheizten Reaktionspartner Amin und Phosgen werden bevorzugt über eine Düsenanordnung der Vermischung und Umsetzung zugeführt. Die Düsenanordnung zur Einführung der Eduktgasströme Amin und Phosgen kann auf verschiedene dem Fachmann bekannte Arten ausgestaltet sein; Beispiele finden sich z. B. in EP 2 199 277 B1, Abschnitt [0017] bis [0019], EP 1 449 826 B1, Abschnitt [0011] bis [0012], EP 1 362 847 B1, Abschnitt [0011] bis [0012], EP 1 526 129 B1, Abschnitt [0009] bis [0011] und EP

1 555 258 B1, Abschnitt [0008] bis [0011].

Neben der bereits erwähnten Möglichkeit, den gasförmigen Strom des primären Amins und den gasförmigen Phosgenstrom zu verdünnen, kann auch ein separater Verdünnungsgasstrom (ein Inertgas wie $N_2$, He, Ar (insbesondere $N_2$) oder die Dämpfe eines inerten Lösungsmittels, bevorzugt ausgewählt aus der Gruppe bestehend aus aliphatischen Kohlenwasserstoffen [vorzugsweise Decahydronaphthalin], aromatischen Kohlenwasserstoffen ohne Halogensubstitution [vorzugsweise Toluol oder Xylol, insbesondere Toluol], aromatischen Kohlenwasserstoffen mit Halogensubstitution [vorzugsweise Chlorbenzol, para-Dichlorbenzol, ortho-Dichlorbenzol, Chlortoluol oder Chlornaphthalin, insbesondere ortho-Dichlorbenzol] und Mischungen der vorgenannten organischen Lösungsmittel) direkt in die Vermischung in Schritt a.3) gefahren werden. In diesem Fall wird dieser Verdünnungsgasstrom bevorzugt auf eine Temperatur im Bereich von 100 °C bis 500 °C, bevorzugt 150 °C bis 450 °C, besonders bevorzugt 150 °C bis 400 °C erhitzt.

Die weitere Umsetzung der in der Mischzone vermischten Reaktionspartner primäres Amin und Phosgen in der Reaktionszone erfolgt bevorzugt adiabatisch. Adiabatische Umsetzung bedeutet, dass auf eine gezielte Abfuhr der entstandenen Reaktionswärme durch ein Wärmeträgermedium verzichtet wird. Daher spiegelt sich die Reaktionsenthalpie - abgesehen von unvermeidbaren Wärmeverlusten - quantitativ in der Temperaturdifferenz von Produkt- und Eduktgasstrom wider. Insbesondere betrifft die Erfindung auch ein Verfahren, bei welchem Schritt a.3) adiabatisch durchgeführt wird und wobei Zusammensetzung und Temperatur des gasförmigen Stroms des primären Amins in Schritt a.1) und des Phosgenstroms in Schritt a.2) jeweils so gewählt werden, dass sich in Schritt a.3) in der Mischzone und in der Reaktionszone eine Temperatur im Bereich von 250 °C bis 450 °C, bevorzugt im Bereich von 270 °C bis 425 °C, besonders bevorzugt im Bereich von 280 °C bis 420 °C, einstellt. Damit ist gemeint, dass die Temperatur an jedem Punkt in der Mischzone und der Reaktionszone in diesem Bereich liegt.

Mischzone und Reaktionszone sind dabei bevorzugt in einer gemeinsamen technischen Vorrichtung zur Durchführung chemischer Reaktionen angeordnet, dem *Reaktor.* Bei dieser Anordnung gehen im Allgemeinen Misch- und Reaktionszone fließend ineinander über, ohne dass - wie bei Einsatz einer separaten Mischapparatur, was grundsätzlich auch möglich ist - eine strenge Abgrenzung beider möglich wäre. Die Reaktionszone nach Vermischung der Reaktanten dient zur Bereitstellung von Verweilzeitraum, um möglichst vollständigen Umsatz sicher zu stellen. Details zum Bau geeigneter Phosgenierungsreaktoren sind dem Fachmann bekannt.

In der Reaktionszone werden Amin und Phosgen rasch zum korrespondierenden Isocyanat umgesetzt, wie beschrieben bevorzugt adiabatisch. Die Reaktion wird bevorzugt so geführt, dass das Amin vor Eintritt in die weiter unten näher beschriebene Quenchzone vollständig umgesetzt ist.

In einem Schritt a.4) erfolgt eine rasche Abkühlung und (bis auf Spurenanteile, die in der Gasphase verbleiben) Verflüssigung des gebildeten gasförmigen Verfahrensprodukts enthaltend das herzustellende Isocyanat ("Quenche") durch Inkontaktbringen mit einer Quenchflüssigkeit in einer Quenchzone. Als Quenchflüssigkeit eignen sich (organische) Lösungsmittel, das herzustellende Isocyanat und Gemische aus dem herzustellenden Isocyanat und einem (organischem) Lösungsmittel, insbesondere Lösungsmittel und Gemische aus dem herzustellenden Isocyanat und einem (organischem) Lösungsmittel. Lösungsmittel für die Quenche sind bevorzugt ausgewählt aus der Gruppe bestehend aus Chlorbenzol, ortho-Dichlorbenzol, para-Dichlorbenzol, den Isomeren des Trichlorbenzols, Toluol, den Isomeren des Xylols und Mischungen der vorgenannten Lösungsmittel. Besonders bevorzugte Lösungsmittel sind Chlorbenzol und Dichlorbenzol, außerordentlich besonders bevorzugt ist ortho-Dichlorbenzol, und zwar insbesondere in Verbindung mit TDI als dem herzustellenden Isocyanat. Das Inkontaktbringen erfolgt bevorzugt durch Eindüsen der Quenchflüssigkeit in den gasförmigen Strom des Reaktionsproduktgemisches.

Möglichkeiten für den Aufbau und den Betrieb einer Quenchzone sind im Prinzip aus dem Stand der Technik bekannt. Die Apparate und Methoden des Standes der Technik können auch im Rahmen der vorliegenden Erfindung eingesetzt werden. Mögliche Ausgestaltungen der Quenchzone sind beispielsweise in EP 1 403 248 A1 und EP 1 935 875 A1 offenbart.

Die Temperatur der in Schritt a.4) eingesetzten Quenchflüssigkeit wird bevorzugt so gewählt, dass sie einerseits hoch genug ist, um das dem Isocyanat entsprechende Carbamoylchlorid in Isocyanat und Chlorwasserstoff zurückzuspalten. (Es ist zwar keineswegs sicher, ob das aus der Flüssigphasenphosgenierung bekannte Zwischenprodukt Carbamoylclorid auch in der Gashasenphosgenierung gebildet wird. Da es jedoch unabhängig davon denkbar ist, dass in der Quenche *verflüssigtes* Isocyanat mit dem vorhandenem Chlorwasserstoffgas teilweise zum Carbamoylchlorid reagiert, sollte die Temperatur der Quenchflüssigkeit hoch genug sein, um diese Reaktion zurückzudrängen.) Andererseits sollen Isocyanat und gegebenenfalls das in dem gasförmigen Aminstrom und / oder gasförmigen Phosgenstrom als Verdünnungsmittel mit verwendete Lösungsmittel weitestgehend kondensieren bzw. sich weitestgehend in dem Lösungsmittel lösen, während überschüssiges Phosgen, Chlorwasserstoff und gegebenenfalls als Verdünnungsmittel mit verwendetes Inertgas die Quenchzone weitestgehend nicht kondensiert bzw. nicht gelöst durchlaufen, sodass die Temperatur der Quenchflüssigkeit auch nicht zu hoch gewählt werden darf. Zur selektiven Gewinnung des Isocyanats aus dem gasförmigen Reaktionsgemisch besonders gut geeignet sind bei einer Temperatur von 50 °C bis 200 °C, vorzugsweise 80 C bis 180 °C gehaltene Quenchflüssigkeiten.

Es ist für den Fachmann aufgrund der physikalischen Daten bei gegebener Temperatur, Druck und Zusammensetzung einfach vorhersagbar, welcher Massenanteil des Isocyanats in der Quenche kondensiert bzw. diese nicht kondensiert durchläuft. Ebenso ist es einfach vorherzusagen, welcher Massenanteil des überschüssigen Phosgens, Chlorwasserstoffs, gegebenenfalls Lösungsmittel und gegebenenfalls als Verdünnungsmittel verwendeten Inertgases die Quenche unkondensiert durchläuft bzw. sich in der Quenchflüssigkeit löst. Das so in der Quenchzone erhaltene Gemisch aus Reaktionsproduktgemisch und Quenchflüssigkeit enthält daher gasförmige Anteile und flüssige Anteile, ist also zweiphasig.

In einem Schritt a.5) wird das in Schritt a.4) anfallende, zweiphasige Gemisch aus Reaktionsproduktgemisch und Quenchflüssigkeit in eine *Sammelzone* zur Phasentrennung geführt.

In einer bevorzugten Ausführungsform sind Mischzone, Reaktionszone, Quenchzone und Sammelzone von oben nach unten in der genannten Reihenfolge in einem aufrecht stehenden, insbesondere konischen oder zylindrischen oder konisch-zylindrischen, Reaktor angeordnet. In dieser Ausführungsform fließt das in Schritt a.4) anfallende Gemisch aus Reaktionsproduktgemisch und Quenchflüssigkeit schwerkraftbedingt (also "automatisch") in die Sammelzone. Bei anderer Anordnung der Sammelzone ist es unter Umständen erforderlich, das Gemisch aus Reaktionsproduktgemisch und Quenchflüssigkeit in die Sammelzone zu pumpen.

In der Sammelzone findet eine Auftrennung des in Schritt a.4) erhaltenen Gemisches aus Reaktionsproduktgemisch und Quenchflüssigkeit in flüssiges, rohes Verfahrensprodukt und ein gasförmiges rohes Verfahrensprodukt statt. Das flüssige rohe Verfahrensprodukt enthält mindestens das herzustellende Isocyanat (und gegebenenfalls als Quenchflüssigkeit eingesetztes Lösungsmittel, gegebenenfalls Nebenprodukte oder nicht abreagierte, über die Reaktionspartner eingetragene Verunreinigungen, gegebenenfalls gelöstes überstöchiometrisch eingesetztes Phosgen sowie gegebenenfalls gelösten Chlorwasserstoff). Das gasförmige rohe Verfahrensprodukt enthält mindestens das Reaktionskoppelprodukt Chlorwasserstoff (und gegebenenfalls überstöchiometrisch eingesetztes Phosgen, gegebenenfalls verdampftes Lösungsmittel, gegebenenfalls Inertgase und gegebenenfalls nicht verflüssigtes herzustellendes Isocyanat). Die flüssige und gasförmige Phase werden der Sammelzone bevorzugt kontinuierlich entnommen. Die so erhaltende Flüssigphase ist in dieser Ausführungsform das Ausgangsmaterial der in Schritt b) durchzuführenden Aufarbeitung, d. h. diese Flüssigphase ist *das herzustellende Isocyanat umfassende flüssige rohe Verfahrensprodukt.*

Nach der Phosgenierung in Schritt a) erfolgt in **Schritt b)** die Aufarbeitung des erhaltenen, das herzustellende Isocyanat umfassende, flüssigen rohen Verfahrensproduktes. Die Aufarbeitung des rohen Isocyanats kann nach allgemein bekannten Methoden erfolgen. Beispiele sind in EP-A-1 413 571, US 2003/0230476 A1 (TDI), und EP 0289 840 B1 (HDI, IDPI und H12-MDI) beschrieben.

Optional werden zunächst gelöstes Phosgen und gelöster Chlorwasserstoff aus dem in Schritt a) erhaltenen flüssigen rohen Verfahrensprodukt in einem separaten Schritt b.1) abgetrennt. Diese Verfahrensführung ist insbesondere dann bevorzugt, wenn die Phosgenierung in Schritt a) in der Flüssigphase durchgeführt wird, weil das in einer Flüssigphasenphosgenierung erhaltene flüssige rohe Verfahrensprodukt tendenziell signifikant höhere Anteile an gelöstem Phosgen und gelöstem Chlorwasserstoff enthält als das in einer Gasphasenphosgenierung erhaltene. Schritt b.1) kann grundsätzlich in jeder dem Fachmann bekannten Art und Weise durchgeführt werden, insbesondere durch Destillation, Absorption oder eine Kombination beider. Weiter unten werden anhand von verschiedenen Varianten mögliche Ausführungsformen gezeigt.

Im Anschluss an Schritt b.1) - oder, insbesondere bei Durchführung des Schritts a) in der Gasphase - unmittelbar im Anschluss an Schritt a), kann sich eine Abtrennung von Lösungsmittel in einem separaten Schritt b.2) anschließen. Schritt b.2) kann in jeder dem Fachmann bekannten Art und Weise durchgeführt werden, insbesondere durch Destillation. Weiter unten werden anhand von verschiedenen Varianten mögliche Ausführungsformen gezeigt.

In Schritt b.3) des erfindungsgemäßen Verfahrens wird das herzustellende Isocyanat durch Destillation isoliert. Dies kann grundsätzlich auf jede dem Fachmann für diesen Zweck bekannte Art und Weise geschehen. Weiter unten werden anhand von verschiedenen Varianten mögliche Ausführungsformen gezeigt.

Für die Ausgestaltung der Aufarbeitung gemäß Schritt b) im Detail sind verschiedene Ausführungsformen möglich. Bevorzugte Varianten sind im Folgenden am Beispiel von TDI geschildert:

## Variante 1

Die Variante 1, die sich besonders dann eignet, wenn Schritt a) in der Flüssigphase durchgeführt wird, ist grundsätzlich beschrieben in Chem System's PERP Report for TDI/MDI (Chem Systems, Process Evaluation Research Planning TDI/MDI 98/99 S8, Tarrytown, N.Y., USA: Chem Systems 1999, S. 27 bis 32). In dieser Variante enthält das flüssige Reaktionsgemisch nach erfolgter destillativer Abtrennung von Chlorwasserstoff und Phosgen (entsprechend dem Schritt b.1) in der Terminologie der vorliegenden Erfindung) noch einen, bezogen auf seine Gesamtmasse, Lösungsmittelanteil von > 50 Massen-%, bevorzugt 51 Massen-% bis 85 Massen-%, besonders bevorzugt 55 Massen-% bis 65 Massen-%. Dieses Gemisch wird einer Lösungsmittelabtrennung (entsprechend dem Schritt b.2) in der Terminologie der vorliegenden Erfindung) zugeführt, wobei zunächst in einem Vorverdampfer ein Lösungsmittel-TDI-Gemisch in eine Lösungsmitteldestillationskolonne abdestilliert wird. In der Lösungsmitteldestillationskolonne wird Lösungsmittel abdestilliert und dem Prozess wieder zugeführt. Der Sumpfstrom dieser Lösungsmitteldestillation enthält, bezogen auf die Gesamtmasse des Sumpfes, neben TDI insbesondere noch bevorzugt 15 Massen-% bis 25 Massen-% Lösungsmittel, bezogen auf die Gesamtmasse dieses Sumpfstroms. Dieser Strom wird in eine so genannte Zwischenkolonne geleitet, in der weiteres Lösungsmittel abdestilliert und das von Lösungsmittel befreite Sumpfprodukt einer letzten Destillationskolonne zur Reinigung des TDI zugeführt wird. Diese wird im Unterdruck betrieben und liefert das gereinigte verkaufsfähige Isocyanat TDI als Destillatstrom (entsprechend dem Schritt b.3) in der Terminologie der vorliegenden Erfindung). Ein Teil des TDI verbleibt im Destillationssumpfstrom dieser letzten Destillationskolonne. Die Aufgaben der Zwischenkolonne und der Destillationskolonne zur TDI-Reinigung können auch, wie in US 2003/0230476 A1, beschrieben in einer Trennwandkolonne zusammengefasst werden, wobei ein Brüdenstrom aus Leichtsiedern und Lösungsmittel, eine Fraktion reines TDI als im Bereich der Trennwand entnommener Destillatstrom sowie ein TDI und höhersiedende Komponenten (Destillationsrückstand) enthaltender Produktstrom als Destillationssumpfstrom erhalten werden. Der Destillationssumpfstrom der Destillationskolonne zur TDI-Reinigung bzw. der Zwischen- und TDI-Reinigungskolonne vereinigenden Trennwandkolonne wird zur Rückgewinnung von TDI aufgearbeitet. Zu diesem Zweck ist es möglich, diesen Strom, wie in Abbildung II.A.5 des genannten PERP-System-Berichts gezeigt, in den Vorverdampfer der Lösungsmitteldestillation zu leiten. Das Sumpfprodukt dieses Vorverdampfers wird dann der Aufarbeitung zur Rückgewinnung von darin enthaltenem TDI geleitet. Die in Chem System's PERP Report for TDI/MDI (Chem Systems, Process Evaluation Research Planning TDI/MDI 98/99 S8, Tarrytown, N.Y., USA: Chem Systems 1999, S. 27 bis 32) in Abbildung II.A.6 gezeigte Behandlung in der *"TDI Residue Processing Facility"* kann durch den Schritt c) der vorliegenden Erfindung ersetzt werden. Da in dieser Ausführungsform das dem Schritt c) zugeführte Ausgangsmaterial infolge der Einspeisung des Destillationssumpfstromes aus dem Schritt b.3) in den Vorverdampfer der Lösungsmittelabtrennung aus Schritt b.2) noch Lösungsmittel enthält (nämlich insbesondere 2,0 Massen-% bis 10 Massen-% Lösungsmittel, bezogen auf die Gesamtmasse dieses Ausgangsmaterials), ist es bevorzugt, den Schritt c.1) durchzuführen und dieses Lösungsmittel dort vor der Trocknung in Schritt c.2) abzutrennen. Selbstverständlich ist es auch möglich, auf die Einspeisung des Destillationssumpfstromes aus Schritt b.3) in den Vorverdampfer zu verzichten und diesen Destillationssumpfstrom stattdessen unmittelbar der Aufarbeitung in Schritt c) zuzuführen.

**Variante 2**

Im Gegensatz zu Variante 1 enthält in dieser Ausführungsform das flüssige Reaktionsgemisch nach erfolgter destillativer Abtrennung von Chlorwasserstoff und Phosgen noch einen, auf seine Gesamtmasse bezogenen, Lösungsmittelanteil von nur $\leq$ 50,0 Massen-%. Dieses Gemisch wird einem Vorverdampfer zugeführt, aus dem ein Lösungsmittel-TDI-Gemisch in eine Destillationskolonne abdestilliert wird. In dieser Variante wird das TDI bereits in der letztgenannten Destillationskolonne vom Lösungsmittel befreit, sodass der Sumpfstrom dieser Destillationskolonne in die TDI-Reinigungskolonne geleitet werden kann, es mithin in dieser Variante eine Kolonne weniger gibt als in Variante 1. Die TDI-Reinigungskolonne wird im Unterdruck betrieben und liefert das gereinigte verkaufsfähige Isocyanat TDI als Destillatstrom. Die Aufgaben der TDI-Reinigungskolonne und der dieser vorgeschalteten Destillationskolonne können auch, wie in EP 1 413 571 A1 beschrieben, in einer Trennwandkolonne zusammengefasst werden, wobei ein Brüdenstrom aus Leichtsiedern und Lösungsmittel, eine Fraktion reines TDI als im Bereich der Trennwand entnommener Destillatstrom sowie ein TDI und höhersiedende Komponenten (Destillationsrückstand) enthaltender Produktstrom als Destillationssumpfstrom erhalten werden. Der Destillationssumpfstrom der TDI-Reinigungskolonne bzw. der Destillationssumpfstrom der die TDI-Reinigungskolonne und der dieser vorgeschalteten Destillationskolonne vereinigenden Trennwandkolonne wird zur Rückgewinnung von TDI aufgearbeitet. Auch in Variante 2 kann diese Aufarbeitung gemäß Schritt c) der vorliegenden Erfindung durchgeführt werden. Zu diesem Zweck ist es möglich, diesen Strom in den oben genannten Vorverdampfer zu leiten. Das Sumpfprodukt dieses Vorverdampfers wird dann der Aufarbeitung zur Rückgewinnung von darin enthaltenem TDI geleitet. Da in dieser Ausführungsform das dem Schritt c) zugeführte Ausgangsmaterial infolge der Einspeisung des Destillationssumpfstromes in den Vorverdampfer der Lösungsmittelabtrennung noch Lösungsmittel enthält (nämlich insbesondere 2,0 Massen-% bis 10 Massen-% Lösungsmittel, bezogen auf die Gesamtmasse dieses Ausgangsmaterials), ist es bevorzugt, den Schritt c.1) durchzuführen und dieses Lösungsmittel dort vor der Trocknung in Schritt c.2) abzutrennen. Selbstverständlich ist es auch möglich, auf die Einspeisung des Destillationssumpfstromes aus Schritt b.3) in den Vorverdampfer zu verzichten und diesen Destillationssumpfstrom stattdessen unmittelbar der Aufarbeitung in Schritt c) zuzuführen.

**Variante 3**

Die Variante 3 umfasst die in den Varianten 2 und 1 beschriebenen Destillationssequenzen, jedoch ohne den jeweils erwähnten Vorverdampfer, der einen flüssigen Sumpfaustrag der Aufarbeitung gemäß Schritt c) zuführt. In diesem Fall wird der Anteil an Destillationsrückstand in den beschriebenen Destillationssequenzen über die flüssigen Mengenströme bis zur jeweiligen letzten TDI-Reinigungskolonne mitgeführt. Dieses Verfahren ist prinzipiell ebenfalls

bekannt (EP 1 717 223 A2). In diesem Fall erfolgt der komplette Austrag des Destillationsrückstands über den Destillationssumpfstrom der letzten Destillationskolonne (die in der Terminologie der vorliegenden Erfindung dem Schritt b.3) zuzuordnen ist). Auch in Variante 3 kann die Aufarbeitung dieses Destillationssumpfstromes gemäß Schritt c) der vorliegenden Erfindung durchgeführt werden.

Variante 4

Diese Variante wird insbesondere eingesetzt, wenn Schritt a) in der Gasphase durchgeführt wird. Da das in einer Gasphasenphosgenierung erhaltene flüssige rohe Verfahrensprodukt gelöstes Phosgen und gelösten Chlorwasserstoff allenfalls in verhältnismäßig (d. h. verglichen mit der Flüssisphasenphosgenierung) geringer Menge enthält, kann auf eine separate Abtrennung von Chlorwasserstoff und Phosgen in Schritt b.1) verzichtet werden. Das flüssige rohe Verfahrensprodukt wird entweder direkt einer Lösungsmittelabtrennung (entsprechend Schritt b.2)) zugeführt, in welcher Lösungsmittel und gegebenenfalls gelöster Chlorwasserstoff sowie gegebenenfalls gelöstes Phosgen destillativ über Kopf abgetrennt werden, oder - wenn der Lösungsmittelanteil hinreichend gering ist - es wird direkt einer TDI-Reinigungskolonne zugeführt. Die TDI-Reinigungskolonne ist in beiden Fällen bevorzugt als Trennwandkolonne ausgestaltet. Leichtsieder (also leichter als TDI siedende Nebenprodukte, gegebenenfalls noch vorhandener Chlorwasserstoff und gegebenenfalls noch vorhandenes Phosgen, gegebenenfalls Lösungsmittel sowie gegebenenfalls Inertgase) werden als Brüden über Kopf abgezogen. Das gereinigte TDI wird als Destillatstrom im Bereich der Trennwand abgeführt. Der anfallende Destillationssumpfstrom enthält den Destillationsrückstand und eine gewisse Menge TDI, die, um den Destillationssumpfstrom verarbeitbar zu halten, nicht abdestilliert wird, sowie gegebenenfalls Spurenanteile von Lösungsmittel. Anstelle einer Trennwandkolonne können selbstverständlich auch zwei in Serie geschaltete Destillationskolonnen ohne Trennwand eingesetzt werden.

In dieser Variante wird die Lösungsmittelabtrennung gemäß Schritt b.2) - sofern durchgeführt - bevorzugt bei einer Temperatur im Bereich von 160 °C bis 200 °C und bei einem Druck im Bereich von 160 mbar bis 220 mbar durchgeführt, wobei sich beide Angaben auf den Sumpf der eingesetzten Destillationskolonne beziehen. Auf diese Weise wird ein Sumpfstrom erhalten, der, bezogen auf seine Gesamtmasse, bevorzugt 9 Massen-% bis 20 Massen-% Lösungsmittel, 79 Massen-% bis 90 Massen-% TDI und 1 bis 5 Massen-% an höher als TDI siedenden Verbindungen enthält.

Die TDI-Reinigung gemäß Schritt b.3) wird, insbesondere bei Durchführung in einer Trennwandkolonne, bevorzugt bei einer Temperatur im Bereich von 160 °C bis 200 °C und bei einem Druck im Bereich von 50 mbar bis 100 mbar durchgeführt, wobei sich beide Angaben auf den Sumpf der eingesetzten Destillationskolonne beziehen. Auf diese Weise wird ein Destillationssumpfstrom erhalten, der, bezogen auf seine Gesamtmasse, bevorzugt 0,00 Massen-% bis 1,00 Massen-% Lösungsmittel, 80,0 Massen-% bis 95,0 Massen-% TDI und 4,00 bis 20,0 Massen-% an höher als TDI siedenden Verbindungen enthält.

Unabhängig von der exakten Ausgestaltung des Schritts b) werden in Schritt b.3) in allen möglichen Verfahrensführungen (mindestens) ein Destillatstrom enthaltend einen ersten Teil des herzustellenden Isocyanats und (mindestens) ein Destillationssumpfstrom enthaltend einen zweiten Teil des herzustellenden Isocyanats und Destillationsrückstand erhalten. Die Aufarbeitung des Destillationssumpfstroms ist Gegenstand von Schritt c) der vorliegenden Erfindung und wird weiter unten noch näher erläutert. Wie bei Variante 1 und 2 erläutert, können mit dem Destillationssumpfstrom aus Schritt b.3) auch noch weitere Sumpfströme dem Schritt c) zugeführt werden.

Neben dem *flüssigen rohen Verfahrensprodukt,* dessen Aufarbeitung zuvor geschildert wurde, wird in Schritt a) auch ein *gasförmiges rohes Verfahrensprodukt* erhalten, das bevorzugt ebenfalls aufgearbeitet wird, und zwar insbesondere, um das gebildete Koppelprodukt Chlorwasserstoff einer wirtschaftlich sinnvollen Verwertung zuzuführen. Das gasförmige rohe Verfahrensprodukt enthält mindestens das Reaktionskoppelprodukt Chlorwasserstoff (und gegebenenfalls überstöchiometrisch eingesetztes Phosgen, gegebenenfalls verdampftes Lösungsmittel, gegebenenfalls Inertgase und gegebenenfalls nicht verflüssigtes herzustellendes Isocyanat). Dieser gasförmige Produktstrom wird bevorzugt einer weiteren Aufarbeitung zugeführt, in der Chlorwasserstoff gereinigt wird. Die gegebenenfalls noch vorhandenen Bestandteile des Gasstroms Phosgen und Lösungsmittel werden voneinander getrennt. Der zurückgewonnene Chlorwasserstoff kann verschiedenen Einsatzmöglichkeiten zugeführt werden, wie zum Beispiel einer Oxychlorierung von Ethylen zu Ethylendichlorid oder einem Recyclingverfahren, welches Chlor, das wieder in den Isocyanatprozess zurückgeführt werden kann, liefert. Zu diesen Recyclingverfahren zählen die katalytische Oxidation von Chlorwasserstoff, z. B. nach dem Deacon-Verfahren, die Elektrolyse von gasförmigem Chlorwasserstoff sowie die Elektrolyse einer wässrigen Lösung von Chlorwasserstoff (Salzsäure). Gegebenenfalls zurückgewonnenes Phosgen wird bevorzugt wieder in Schritt a) eingesetzt. Gegebenenfalls zurückgewonnenes Lösungsmittel wird bevorzugt ebenfalls wieder in Schritt a) eingesetzt (bspw. als Lösungsmittel für die Reaktionspartner primäres Amin und Phosgen einer Flüssigphasenphosgenierung oder in der Quenche einer Gasphasenphosgenierung).

Die Aufarbeitung des in Schritt b.3) erhaltenen Destillationssumpfstromes ist Gegenstand von **Schritt c) (Schritte c.1) und c.2)** des erfindungsgemäßen Verfahrens. Dieser Destillationssumpfstrom besteht neben Anteilen des herzustellenden Isocyanats (die möglichst vollständig zurückgewonnen werden sollen) sowie gegebenenfalls Lösungsmittelanteilen aus dem Destillationsrückstand.

Bevorzugt ist es, den Destillationssumpfstrom zunächst in einem Schritt c.1) vorzukonzentrieren, d. h. das herzustellende Isocyanat bereits teilweise durch Verdampfung abzutrennen, ohne das der verbleibende flüssige Strom fest wird. Diese Vorkonzentrierung durch partielle Verdampfung kann grundsätzlich in allen dem Fachmann bekannten Verdampfern erfolgen. Besonders bevorzugt wird Schritt c.1) in einem Verdampfer ausgewählt aus der Gruppe bestehend aus Dünnschichtverdampfern, Kletterverdampfem, Fallfilmverdampfern, Langrohrverdampfern, Wendelrohrverdampfern, Zwangsumlaufentspannungsverdampfer und einer Kombination dieser Apparate durchgeführt. Dabei sind Fallfilmverdampfer besonders bevorzugt. Es ist auch möglich, mehrere Verdampfer in Serie zu schalten. Bevorzugt erfolgt die Vorkonzentrierung gemäß Schritt c.1) bei einer Temperatur im Bereich von 120 °C bis 180 °C und bei einem Druck im Bereich von 20 mbar($_{abs.}$) bis 60 mbar($_{abs.}$), besonders bevorzugt bei einer Temperatur im Bereich von 130 °C bis 175 °C und bei einem Druck im Bereich von 25 mbar($_{abs.}$) bis 45 mbar($_{abs}$). Schritt c.1) kann kontinuierlich oder diskontinuierlich durchgeführt werden. Die kontinuierliche Verfahrensführung ist bevorzugt.

In Schritt c.2) erfolgt nun erfindungsgemäß die Trocknung des in Schritt c.1) erhaltenen, an herzustellendem Isocyanat abgereicherten, vorkonzentrierten flüssigen Stroms bzw. - bei Verzicht auf Schritt c.1) - des in Schritt b.3) erhaltenen Destillationssumpfstromes. Diese Trocknung wird bei einer Temperatur im Bereich von 150 °C bis 500 °C, bevorzugt im Bereich von 185 °C bis 300 °C, besonders bevorzugt im Bereich von 200 °C bis 270 °C, in einer Trocknungsvorrichtung durchgeführt. Für den Schritt c.2) geeignete Trocknungsvorrichtungen sind bevorzugt ausgewählt aus der Gruppe bestehend aus beheizten, produktumschaufelnden Vakuumtrocknern mit horizontaler Welle (bevorzugt Knetertrockner, Paddeltrockner, Schaufeltrockner; wobei jeder der genannten Trockner genau eine Welle oder mehrere Wellen, insbesondere zwei Wellen, aufweisen kann), Drehrohren, Scheibentrocknern, Bandtrocknern und Granulierschnecken. Bei der Trocknung wird das herzustellende Isocyanat verdampft und zurückgewonnen. Es verbleibt ein Feststoff, der nahezu ausschließlich aus Destillationsrückstand besteht und das herzustellende Isocyanat allenfalls noch in Spuren enthält (bevorzugt maximal 1,0 Massen-% des herzustellenden Isocyanats, besonders bevorzugt maximal 0,1 Massen-% des herzustellenden Isocyanats, jeweils bezogen auf die Gesamtmasse des in Schritt c.2) anfallenden Feststoffes). Der Feststoff wird bevorzugt kontinuierlich aus der Trocknungsvorrichtung ausgetragen.

Erfindungsgemäß wird der auf die Gesamtmasse des in die Trocknung gemäß Schritt c.2) eingetragenen Destillationsrückstandes bezogene Massenanteil an Carbodiimidgruppen-haltigen Verbindungen auf einen Wert von mindestens 15 %, bevorzugt von mindestens 20 %, besonders bevorzugt von mindestens 30 % eingestellt. Dies kann wie folgt geschehen:

**Fall A:** Die Verfahrensbedingungen in Schritt a) und/oder in Schritt b) und/oder - sofern durchgeführt - in Schritt c.1) begünstigen die Bildung von Carbodiimidgruppen-haltigen Verbindungen und werden insbesondere so gewählt, dass die über das Ausgangsmaterial von Schritt c.2) in diesen Schritt eingetragene Masse an Carbodiimidgruppen-haltigen Verbindungen so groß ist, dass die erfindungsgemäßen Anforderungen an den minimalen Massenanteil an Carbodiimidgruppen-haltigen im Destillationsrückstand erfüllt werden.

Fall A ist insbesondere dann bevorzugt, wenn die Phosgenierung gemäß Schritt a) in der Flüssigphase erfolgt. Die Bildung von Carbodiimidgruppen-haltigen Verbindungen wird durch hohe Temperaturen begünstigt. Daher kann durch eine Temperaturerhöhung und/oder durch eine Erhöhung der Verweilzeit - jeweils im Vergleich zum Betriebszustand mit nicht ausreichender Carbodiimidbildung- in Schritt a) und/oder in Schritt b) und/oder - sofern durchgeführt - in Schritt c.1) der Anteil an Carbodiimidgruppen-haltigen Verbindungen gesteigert werden. Um wie viel die Temperatur und/oder die Verweilzeit erhöht werden müssen, ist für den Fachmann durch einfache Vorversuche leicht zu ermitteln.

**Fall B:** Carbodiimidgruppen-haltige Verbindungen werden in einem separaten Prozess aus dem herzustellen Isocyanat hergestellt und dem Schritt c.2) zugeführt, insbesondere in solcher Menge, dass die erfindungsgemäßen Anforderungen erfüllt werden. Dieser Fall ist insbesondere dann bevorzugt, wenn Schritt a) in der Gasphase durchgeführt wird.

Carbodiimidgruppen-haltige Verbindungen werden in einer Ausführungsform der Erfindung hergestellt, indem ein das herzustellende Isocyanat enthaltende Verfahrensprodukt aus dem erfindungsgemäßen Herstellverfahren (insbesondere ein Teil des in Schritt b.3) erhaltenen Destillatstroms oder - sofern durchgeführt - ein Teil des in Schritt b.2) anfallenden an Phosgen, Chlorwasserstoff und Lösungsmittel abgereicherten flüssigen Verfahrensprodukts) bei erhöhter Temperatur, insbesondere bei einer Temperatur im Bereich von 200 °C bis 270 °C für einen Zeitraum von insbesondere 30 Minuten bis 10 Stunden getempert wird. Anstelle einer rein thermischen Behandlung kann die Carbodiimidisierung auch katalytisch induziert werden. Geeignete Katalysatoren sind insbesondere Katalysatoren vom Phospholinoxid-Typ. Unter Katalysatoren vom Phospholinoxid-Typ werden im Rahmen der vorliegenden Erfindung am Phosphoratom substituierte 1-Oxo-Phosphacyclopentene, *cyclo*-$C_4H_6P(O)R$, verstanden, wobei der Substituent R für einen gesättigten oder ungesättigten, optional substituierten, insbesondere Halogen-substituierten, organischen Rest, insbesondere Methyl oder Phenyl, steht. Die Katalysatoren vom Phospholinoxid-Typ sind beispielsweise aus EP-A-0 515 933 und US-A-6,120,699 bekannt. Typische Beispiele dieser Katalysatoren sind insbesondere die aus dem Stand der Technik bekannten Gemische der Phospholinoxide der Formel:

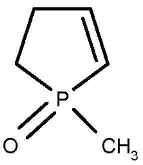

und

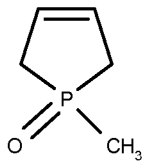

Die jeweils notwendige Katalysatormenge kann der Fachmann in Vorversuchen auf einfache Weise ermitteln. Die Carbodiimidisierung in Gegenwart eines Katalysators wird bevorzugt bei einer Temperatur im Bereich von 50 °C bis 150 °C, vorzugsweise im Bereich von 60 °C bis 100 °C, durchgeführt. Der Anteil des in Schritt b.3) erhaltenen Destillatstroms oder - sofern durchgeführt - des des in Schritt b.2) anfallenden an Phosgen, Chlorwasserstoff und Lösungsmittel abgereicherten flüssigen Verfahrensprodukts, der wie beschrieben einer thermisch oder katalytisch induzierten Carbodiimidisierung unterworfen wird, kann vom Fachmann durch Routineexperimente einfach bestimmt werden. Der verbleibende Teil des in Schritt b.3) erhaltenen Destillatstroms wird in der üblichen Weise (z. B. Verkauf, Umsetzung zu Polyurethanen, Umsetzung zu Präpolymeren, Abmischung mit anderen Isocyanaten zu Blends und dgl. mehr) weiter verwendet. Der verbleibende Teil des gegebenenfalls verwendeten in Schritt b.2) anfallenden an Phosgen, Chlorwasserstoff und Lösungsmittel abgereicherten flüssigen Verfahrensprodukts wird in Schritt b.3) destilliert. Das nach beendeter Carbodiimidisierung erhaltene Verfahrensprodukt wird mit dem in Schritt c.1) erhaltenen vorkonzentrierten flüssigen Strom bzw. - bei Verzicht auf Schritt c.1) - dem in Schritt b.3) erhaltenen Destillationssumpfstrom vor der Zuführung desselben in die Trocknungsvorrichtung aus Schritt c.2) vermischt oder das nach beendeter Carbodiimidisierung erhaltene Verfahrensprodukt wird dieser Trocknungsvorrichtung über einen separaten Einlaufstutzen zugeführt.

In einer anderen Ausführungsform wird carbodiimidisiertes Isocyanat aus einem anderen Herstellungsprozess des gleichen herzustellenden Isocyanats entnommen. Es ist beispielsweise möglich, den Destillationssumpfstrom aus Schritt b.3) oder den vorkonzentrierten flüssigen Strom aus Schritt c.1) einer Produktionsanlage, in welcher die Produktionsbedingungen die Bildung von Carbodiimidgruppen-haltigen Verbindungen begünstigen, ganz oder teilweise mit den entsprechenden Strömen einer anderen Produktionsanlage, in welcher die Produktionsbedingungen der Bildung von Carbodiimidgruppen-haltigen Verbindungen abträglich sind, zu vermischen oder direkt dem Schritt c.2) dieser letztgenannten Produktionsanlage zuzuführen.

Sofern die Trocknung in Schritt c.2) nicht gemäß einer der beiden im Folgenden noch näher geschilderten Ausführungsformen für den Fall C durchgeführt wird, wird in der Trocknungsvorrichtung aus Schritt c.2) für die Dauer dieses Schrittes bevorzugt ein Druck im Bereich von 10 mbar($_{abs.}$) bis 350 mbar($_{abs.}$), besonders bevorzugt im Bereich von 20 mbar($_{abs.}$) bis 200 mbar($_{abs.}$), ganz besonders bevorzugt im Bereich von 30 mbar($_{abs.}$) bis 100 mbar($_{abs.}$) eingehalten. Dies gilt demnach für jede denkbare Kombination der Fälle A und B.

**Fall C:** Carbodiimidgruppen-haltige Verbindungen werden in Schritt c.2) *in situ* gebildet, insbesondere in ausreichender Menge, um die erfindungsgemäßen Anforderungen zu erfüllen. Dies kann auf verschiedene Weisen geschehen. Zwei bevorzugte Ausführungsformen werden im Folgenden geschildert:

In einer ersten Ausführungsform wird die Trocknung gemäß Schritt c.2) zunächst in einem ersten Teilschritt c.2.1) bei relativ hohem Druck durchgeführt, bevor zu einem späteren Zeitpunkt in einem zweiten Teilschritt c.2.2) der Druck erniedrigt wird. Auf diese Weise wird es ermöglicht, dass vor dem Beginn des "eigentlichen" Trockenvorgangs im zweiten Teilschritt mit niedrigem Druck Carbodiimidgruppen-haltige Verbindungen in ausreichender Menge gebildet werden. Bei kontinuierlicher Durchführung des Schrittes c.2) wird bevorzugt Teilschritt c.2.2) in einem eigenen Apparat durchgeführt, der dem Apparat für Teilschritt c.2.1) nachgeschaltet ist. Der erste Teilschritt des Schrittes c.2) dieser Ausführungsform wird bevorzugt bei einem Druck im Bereich von > 750 mbar($_{abs.}$) bis 1013 mbar($_{abs.}$) und bei einer Temperatur im Bereich von 200 °C bis 270 °C für einen Zeitraum von insbesondere 30 Minuten bis 10 Stunden durchgeführt. Im Anschluss wird der Druck im zweiten Teilschritt gesenkt auf einen Wert im Bereich von 10 mbar($_{abs.}$) bis 250 mbar($_{abs}$), bevorzugt 20 mbar($_{abs}$) bis 200 mbar($_{abs}$), besonders bevorzugt 30 mbar($_{abs}$) bis 100 mbar($_{abs}$). Die Temperatur liegt bevorzugt im gleichen Bereich wie im ersten Teilschritt (also im Bereich von 200 °C bis 270 °C) und wird besonders bevorzugt gegenüber der Temperatur im ersten Teilschritt nicht verändert. Der zweite Teilschritt wird bevorzugt für einen Zeitraum von 30 Minuten bis 360 Minuten durchgeführt. Der Übergang von der Phase hohen Drucks zur Phase niedrigen Drucks kann auch kontinuierlich unter langsamer Druckerniedrigung erfolgen.

In einer zweiten Ausführungsform wird während des (ganzen) Schrittes c.2) ein Druck eingehalten, der sowohl eine ausreichende Bildung von Carbodiimidgruppen-haltigen Verbindungen als auch das Trocknen des Destillationssumpfstromes gestattet. In dieser Ausführungsform wird Schritt c.2) bevorzugt bei einem Druck im Bereich von > 250 mbar($_{abs}$) bis 750 mbar($_{abs}$), bevorzugt 300 mbar$_{(abs.)}$ bis 650 mbar$_{(abs.)}$, besonders bevorzugt 450 mbar$_{(abs.)}$ bis 550 mbar($_{abs}$). Die Formulierung *"während des (ganzen) Schrittes c.2)"* umfasst dabei selbstverständlich auch Ausführungsformen, in denen der angestrebte Zieldruck in den genannten Bereichen nicht sofort, sondern etwa erst nach einer Aufheizphase eingeregelt wird. Die Temperatur liegt (für alle Druckbereiche) bevorzugt im Bereich von 200 °C bis 270

°C. In dieser Ausführungsform wird Schritt c.2) bevorzugt für einen Zeitraum von 30 Minuten bis 600 Minuten durchgeführt.

Im Fall C ist ein gewisser Ausbeuteverlust an herzustellendem Isocyanat durch die *In situ*-Carbodiimidisierung unvermeidbar. Wenn hierdurch jedoch eine betriebsstabile Trocknung erreicht werden kann, überwiegen die Vorteile diesen unvermeidbaren Nachteil.

In einer bevorzugten Ausführungsform wird in Schritt c.2) ein Trennmittel zugesetzt, um die Bildung nicht klebriger getrockneter Rückstandspartikel zu erleichtern. Dies gilt unabhängig davon, ob Fall A, B oder C vorliegt. Solche Trennmittel können Zusatzstoffe sein, die bei der Temperatur im Reaktor nicht klebrig sind, also bei Aufeinandertreffen nicht zu größeren Agglomeraten verkleben, mit dem Isocyanat nicht reagieren und die Polymerisationsreaktion der oligomeren Bestandteile des ursprünglichen Destillationsrückstands nicht behindern. Bevorzugt ist das Trennmittel ausgewählt aus der Gruppe bestehend aus Bitumen, Talkum, Kreide, anorganischen Pigmenten und vollständig durchgetrocknetem Rückstand. Unter den *anorganischen Pigmenten* sind insbesondere Titandioxid, Eisenoxide, Aluminiumoxid und andere Metalloxide bevorzugt. *Vollständig durchgetrockneter Rückstand* meint das in der Trocknungsvorrichtung von Schritt c.2) erhaltene feste Verfahrensprodukt. Zweckmäßigerweise kann nämlich ein Teil des in Schritt c.2) durch Trocknung gewonnenen Feststoffes als Trennmittel in den Trocknungsprozess zurückgeführt werden. Dieser zurückgeführte Anteil kann zuvor noch fein vermahlen werden.

Die Trocknung in Schritt c.2) kann - unabhängig davon, ob Fall A, B oder C vorliegt, diskontinuierlich oder kontinuierlich durchgeführt werden. Bei diskontinuierlicher Fahrweise werden zweckmäßigerweise mehrere Trocknungsvorrichtungen parallel betrieben, um eine Produktionsunterbrechung infolge der notwendigen Entnahme des gebildeten festen Verfahrensprodukts nach beendeter Trocknung zu vermeiden. Bei kontinuierlicher Verfahrensführung, die insbesondere im großtechnischen Maßstab zweckmäßiger ist, wird das gebildete feste Verfahrensprodukt kontinuierlich durch geeignete Austragseinrichtungen (z. B. Schneckenförderer, Paddelförderer, Überlauf über ein Wehr oder Schwerkraftförderung) aus der Trocknungsvorrichtung ausgetragen, insbesondere in Form eines Granulats oder Pulvers. Der auf diese Weise in Schritt c.2) zurückgewonnene Anteil des herzustellenden Isocyanats wird bevorzugt teilweise bis vollständig, bevorzugt vollständig, mit dem in Schritt b) als Destillatstrom erhaltenem Teil der herzustellendem Isocyanats vereinigt und der weiteren Anwendung zugeführt. Es ist ebenfalls möglich, den in Schritt c.2) gewonnenen Anteil des herzustellenden Isocyanats an einer anderen Stelle dem Prozess zuzuführen, z. B. in Schritt b), insbesondere in den Zulauf oder in den Destillationssumpfstrom der in Schritt b.3) eingesetzten Destillationskolonne (bei mehreren in Serie geschalteten Destillationskolonnen bevorzugt in den Zulauf oder in den Destillationssumpfstrom der letzten Destillationskolonne). Mit dem gegebenenfalls in Schritt c.1) zurückgewonnenen Anteil an herzustellendem Isocyanat kann auf die gleiche Weise verfahren werden. Bevorzugt werden die in Schritt c.1) und Schritt c.2) gewonnenen Anteile an herzustellendem Isocyanat vereinigt.

## Beispiele

Die folgenden Beispiele wurden in einer Trocknungsvorrichtung der Fa. List (DTB 6500) aus dem Jahre 1977 durchgeführt. Bei dieser Maschine handelt es sich um einen Einwellenkneter mit einem Volumen von ca. 6,5 L. Der elektrische Antrieb hat eine Leistung von max. 7,5 KW; die Drehzahl der Welle kann variabel eingestellt werden. Der Arbeitsraum des Knetertrockners ist mit einem Doppelmantel ausgestattet und wird mit Öl beheizt; die Rücklauftemperatur des Öls ist wird gemessen und angezeigt. Die während der Trocknung entstehenden Brüden werden in einem wassergekühlten Wärmeaustauscher kondensiert und in einer skalierten Vorlage aufgefangen. Das Vakuum wird mittels einer Membranpumpe erzeugt und über einen Vakuumkonstanthalter geregelt. Der Trocknungsschritt (c.)2) wurde diskontinuierlich durchgeführt. Prozentangaben sind Massen-% bezogen auf die Gesamtmasse des jeweiligen Verfahrensproduktes.

### Beispiel 1 (Vergleich):

Der in einem der letzten Destillationskolonne der Aufarbeitung des flüssigen rohen Verfahrensprodukts einer Phosgenierung nachgeschalteten Fallfilmverdampfer erhaltene, vorkonzentrierte flüssige Strom wurde gemeinsam mit 2,8 Massen-% Bitumen, bezogen auf die Masse des in diesem vorkonzentrierten flüssigen Strom enthaltenen Destillationsrückstands, in der Trocknungsvorrichtung bei 235 °C (Ölrücklauftemperatur) und 40 $mbar_{(abs.)}$ getrocknet. Der zu trocknende vorkonzentrierte flüssige Strom hatte folgende Zusammensetzung:

Destillationsrückstand: 32,6 %,

Carbodiimidgehalt in diesem Destillationsrückstand: 2,8 %.

Der Versuchsverlauf ist in FIG. 1 graphisch dargestellt. Bei der Trocknung kam es zu einem starken Drehmomentanstieg der Welle der Trocknungsvorrichtung, erkennbar an dem Maximum in der Leistungskurve und der großen

Fläche unterhalb dieses Maximums. Durch die dadurch eingetragene Energie stieg die Temperatur zeitweise sogar über die Ölrücklauftemperatur an. Es wurden 98,7 % des im zu trocknenden vorkonzentrierten flüssigen Strom enthaltenden TDI zurückgewonnen. Hieraus errechnet sich nach Messmethode III der Massenanteil an Carbodiimidgruppenhaltigen Verbindungen im Destillationsrückstand im Sinne der vorliegenden Erfindung zu 5,2 %. Es kam also in diesem Beispiel grundsätzlich zu einer *in situ*-Carbodiimidisierung, jedoch in viel zu geringem Ausmaß.

**Beispiel 2 (erfindungsgemäß):**

Der in einem der letzten Destillationskolonne der Aufarbeitung des flüssigen rohen Verfahrensprodukts einer Phosgenierung nachgeschalteten Fallfilmverdampfer erhaltene, vorkonzentrierte flüssige Strom wurde gemeinsam mit 2,8 Massen-% Bitumen, bezogen auf die Masse des in diesem vorkonzentrierten flüssigen Strom enthaltenen Destillationsrückstands, in der Trocknungsvorrichtung bei 233 °C (Ölrücklauftemperatur) und 40 $mbar_{(abs.)}$ getrocknet. Der zu trocknende vorkonzentrierte flüssige Strom hatte folgende Zusammensetzung:

Destillationsrückstand: 30,0 %,

Carbodiimidgehalt in diesem Destillationsrückstand: 21,8 %.

Der Versuchsverlauf ist in FIG. 2 graphisch dargestellt. Bei der Trocknung kam es zu keinem Drehmomentanstieg der Welle der Trocknungsvorrichtung, erkennbar an dem horizontalen Verlauf der Leistungskurve. Außerdem wurde der mechanische Energieeintrag deutlich reduziert, so dass es zu keinem Anstieg der Temperatur oberhalb der Öl-Rücklauftemperatur kam, sondern sich die Temperatur asymptotisch der Öl-Rücklauftemperatur annäherte. Es wurden > 99 % des im zu trocknenden vorkonzentrierten flüssigen Strom enthaltenden TDI zurückgewonnen.

**Beispiel 3 (erfindungsgemäß):**

Der Versuch wurde durchgeführt wie in Beispiel 1 jedoch mit dem Unterschied, dass der vorkonzentrierte flüssige Strom aus dem Fallfilmverdampfer mit dem Bitumenzusatz in die Trocknungsrichtung eingefüllt und 3 Stunden bei 230 °C und 800 $mbar_{(abs.)}$ thermisch behandelt wurde. Anschließend wurde der Druck in der Trocknungseinrichtung langsam abgesenkt und der vorkonzentrierte flüssige Strom in der Trocknungsvorrichtung bei 235 °C (Ölrücklauftemperatur) und 40 $mbar_{(abs.)}$ getrocknet.

Der Versuchsverlauf ist in FIG. 3 graphisch dargestellt. Bei der Trocknung kam es zwar noch zu einem starken Drehmomentanstieg der Welle der Trocknungsvorrichtung, jedoch war dieser Anstieg sehr kurzzeitig. Zeitgleich wurde ein steiler Temperaturanstieg beobachtet, der jedoch durch das Umbrechen in die feste Phase gestoppt wurde. Dadurch wurde der mechanische Energieeintrag deutlich reduziert, so dass es zu keinem weiteren Anstieg der Temperatur oberhalb der Öl-Rücklauftemperatur kam. Es wurden 87,2 % des im zu trocknenden vorkonzentrierten flüssigen Strom enthaltenden TDI zurückgewonnen. Hieraus errechnet sich nach Messmethode III der Massenanteil an Carbodiimidgruppen-haltigen Verbindungen im Destillationsrückstand im Sinne der vorliegenden Erfindung zu 25,9 %.

**Beispiel 4 (erfindungsgemäß):**

Durch 12-tündige thermische Behandlung von TDI bei 220 °C wurde eine TDI-Carbodiimid-Mischung mit einem auf die Gesamtmasse bezogenen Carbodiimidanteil von ~15 % erzeugt. Aus dieser Mischung wurde TDI destillativ entfernt und der Carbodiimidanteil so auf 46,5 % erhöht. Diese Mischung wurde nun mit dem vorkonzentrierten flüssigen Strom aus Beispiel 1 gemischt und gemeinsam mit 2,6 Massen-% Bitumen, bezogen auf die Masse des im vorkonzentrierten flüssigen Strom enthaltenen Destillationsrückstands, in der Trocknungsvorrichtung bei 234 °C (Ölrücklauftemperatur) und 40 $mbar_{(abs.)}$ getrocknet. Der zu trocknende abgemischte, vorkonzentrierte flüssige Strom hatte folgende Zusammensetzung:

Destillationsrückstand 34,6 %,

Carbodiimidgehalt in diesem Destillationsrückstand 20,0 %.

Der Versuchsverlauf ist in FIG. 4 graphisch dargestellt. Bei der Trocknung kam es zu einem kurzen Drehmomentanstieg der Welle der Trocknungsvorrichtung, der deutlich geringer ausgeprägt war als in Beispiel 1. Auch wurde das Überschwingen der Temperatur über die Ölrücklauftemperatur nicht beobachtet. Es wurden > 99 % des im zu trocknenden vorkonzentrierten flüssigen Strom enthaltenden TDI zurückgewonnen.

**Beispiel 5 (erfindungsgemäß):**

Durch 12-stündige thermische Behandlung von TDI bei 220 °C wurde eine TDI-Carbodiimid-Mischung mit einem auf die Gesamtmasse bezogenen Carbodiimidanteil von 15 % erzeugt. Aus dieser Mischung wurde TDI destillativ entfernt und der Carbodiimidanteil so auf 46,5 % erhöht. Diese Mischung wurde nun mit dem vorkonzentrierten flüssigen Strom aus Beispiel 1 gemischt und gemeinsam mit 2,6 Massen-% Bitumen, bezogen auf die Masse des im vorkonzentrierten flüssigen Strom enthaltenen Destillationsrückstands, in der Trocknungsvorrichtung bei 234 °C (Ölrücklauftemperatur) und 40 mbar($_{abs.}$) getrocknet. Der zu trocknende abgemischte, vorkonzentrierte flüssige Strom hatte folgende Zusammensetzung:

Destillationsrückstand 35,7 %,

Carbodiimidgehalt in diesem Destillationsrückstand 30,9 %.

Der Versuchsverlauf ist in FIG. 5 graphisch dargestellt. Bei der Trocknung kam es zu einem kurzen Drehmomentanstieg der Welle der Trocknungsvorrichtung, der deutlich geringer ausgeprägt war wie im Beispiel 4. Auch wurde das Überschwingen der Temperatur über die Ölrücklauftemperatur nicht beobachtet. Es wurden > 99 % des im zu trocknenden vorkonzentrierten flüssigen Strom enthaltenden TDI zurückgewonnen.

**Beispiel 6 (erfindunsssemäß**

Der in einem der letzten Destillationskolonne der Aufarbeitung des flüssigen rohen Verfahrensprodukts einer Phosgenierung nachgeschalteten Fallfilmverdampfer erhaltene vorkonzentrierte flüssige Strom wurde gemeinsam mit 2,8 Massen-% Bitumen, bezogen auf die Masse des im vorkonzentrierten flüssigen Strom enthaltenen Destillationsrückstands, in der Trocknungsvorrichtung bei 258 °C (Ölrücklauftemperatur) und 460 mbar($_{abs}$) getrocknet. Der zu trocknende vorkonzentrierte flüssige Strom hatte folgende Zusammensetzung:

Destillationsrückstand: 32,6 %,

Carbodiimidgehalt in diesem Destillationsrückstand: 2,8 %.

Der Versuch wurde durchgeführt wie in Beispiel 1 jedoch mit dem Unterschied, dass der vorkonzentrierte flüssige Strom in der Trocknungsvorrichtung bei 258 °C (Ölrücklauftemperatur) und 460 mbar($_{abs.}$) getrocknet wurde.

Der zu trocknende vorkonzentrierte flüssige Stromwurde in die vorgeheizte Trocknungsvorrichtung gesaugt. Beim Erreichen von 220 °C in der Trocknungsvorrichtung wurde das Vakuum auf den Zielwert von 460 mbar($_{abs.}$) eingeregelt. Der Versuchsverlauf ist in FIG. 6 graphisch dargestellt. Bei der Trocknung kam es zu einem keinem Drehmomentanstieg der Welle der Trocknungsvorrichtung, erkennbar an dem horizontalen Verlauf der Leistungskurve. Außerdem wurde der mechanische Energieeintrag deutlich reduziert, so dass es zu keinem Anstieg der Temperatur oberhalb der Öl-Rücklauftemperatur kam, sondern sich die Temperatur asymptotisch der Öl-Rücklauftemperatur annäherte. Es wurden 64,6 % des im zu trocknenden vorkonzentrierten flüssigen Strom enthaltenden TDI zurückgewonnen. Hieraus errechnet sich nach Messmethode III der Massenanteil an Carbodiimidgruppen-haltigen Verbindungen im Destillationsrückstand im Sinne der vorliegenden Erfindung zu 66,7 %.

**Beispiel 7 (erfindungsgemäß**

Der Versuch wurde durchgeführt wie in Beispiel 6 jedoch mit dem Unterschied, dass der vorkonzentrierte flüssige Strom in der Trocknungsvorrichtung bei 258 °C (Ölrücklauftemperatur) und 300 mbar($_{abs.}$) getrocknet wurde. Das Vakuum wurde beim Erreichen einer Temperatur von 230 °C in der Trocknungsvorrichtung auf den Zielwert von 300 mbar($_{abs.}$) eingeregelt.

Der Versuchsverlauf ist in FIG. 7 graphisch dargestellt. Bei der Trocknung kam es zu einem keinem Drehmomentanstieg der Welle der Trocknungsvorrichtung, erkennbar an dem horizontalen Verlauf der Leistungskurve. Außerdem wurde der mechanische Energieeintrag deutlich reduziert, so dass es zu keinem Anstieg der Temperatur oberhalb der Öl-Rücklauftemperatur kam, sondern sich die Temperatur asymptotisch der Öl-Rücklauftemperatur annäherte. Es wurden 82,2 % des im zu trocknenden vorkonzentrierten flüssigen Strom enthaltenden TDI zurückgewonnen. Hieraus errechnet sich nach Messmethode III der Massenanteil an Carbodiimidgruppen-haltigen Verbindungen im Destillationsrückstand im Sinne der vorliegenden Erfindung zu 34,9 %.

**Beispiel 8 (erfindungsgemäß**

Der Versuch wurde durchgeführt wie in Beispiel 6 jedoch mit dem Unterschied, dass der in der Trocknungsvorrichtung bei 278 °C (Ölrücklauftemperatur) und 300 $mbar_{(abs.)}$ getrocknet wurde. Das Vakuum wurde beim Erreichen einer Temperatur von 250 °C in der Trocknungsvorrichtung auf den Zielwert von 300 mbar($_{abs}$.) eingeregelt.

Der Versuchsverlauf ist in FIG. 8 graphisch dargestellt. Bei der Trocknung kam es zu einem keinem Drehmomentanstieg der Welle der Trocknungsvorrichtung, erkennbar an dem horizontalen Verlauf der Leistungskurve. Außerdem wurde der mechanische Energieeintrag deutlich reduziert, so dass es zu keinem Anstieg der Temperatur oberhalb der Öl-Rücklauftemperatur kam, sondern sich die Temperatur asymptotisch der Öl-Rücklauftemperatur annäherte. Es wurden 80,2 % des im zu trocknenden vorkonzentrierten flüssigen Strom enthaltenden TDI zurückgewonnen. Hieraus errechnet sich nach Messmethode III der Massenanteil an Carbodiimidgruppen-haltigen Verbindungen im Destillationsrückstand im Sinne der vorliegenden Erfindung zu 38,5 %.

**Patentansprüche**

1. Verfahren zur Herstellung eines Isocyanats durch Phosgenierung des zu dem herzustellenden Isocyanat korrespondierenden primären Amins unter Erhalt eines das herzustellende Isocyanat umfassenden, flüssigen rohen Verfahrensprodukts,
umfassend die Aufarbeitung dieses flüssigen rohen Verfahrensprodukts unter Erhalt eines Destillationssumpfstromes bestehend aus

    • dem herzustellenden Isocyanat,
    • gegebenenfalls Leichtsiedern, das sind Stoffe oder azeotrop siedende Gemische von Stoffen, deren Siedepunkt geringer ist als der des herzustellenden Isocyanats, oder, sofern das herzustellende Isocyanat als Isomerengemisch vorliegt, geringer ist als der des am niedrigsten siedenden Isomers des herzustellenden Isocyanats, und
    • Destillationsrückstand;

    weiterhin umfassend die Aufarbeitung dieses Destillationssumpfstromes, wobei diese Aufarbeitung folgende Schritte umfasst:

    1) optionale Vorkonzentrierung des Destillationssumpfstroms in einem Verdampfer durch partielle Verdampfung des in dem Destillationssumpfstrom enthaltenen herzustellenden Isocyanats, wobei ein an herzustellendem Isocyanat abgereicherter vorkonzentrierter flüssiger Strom erhalten wird;
    2) Trocknung des Destillationssumpfstroms oder des in Schritt 1) erhaltenen vorkonzentrierten, an herzustellendem Isocyanat abgereicherten, flüssigen Stroms in einer Trocknungsvorrichtung bei einer Temperatur im Bereich von 150 °C bis 500 °C, bevorzugt im Bereich von 185 °C bis 300 °C, besonders bevorzugt im Bereich von 200 °C bis 270 °C, wobei herzustellendes Isocyanat unter Bildung eines festen Verfahrensprodukts verdampft und zurückgewonnen wird, wobei der auf die Gesamtmasse des der Trocknungsvorrichtung zugeführten Destillationsrückstandes bezogene Massenanteil an Carbodiimidgruppen-haltigen Verbindungen auf einen Wert von mindestens 15 %, bevorzugt mindestens 20 %, besonders bevorzugt mindestens 30 %, eingestellt wird.

2. Verfahren gemäß Anspruch 1, bei welchem die Phosgenierung in der Flüssigphase in Gegenwart eines Lösungsmittels durchgeführt wird.

3. Verfahren gemäß Anspruch 1, bei welchem die Phosgenierung in der Gasphase durchgeführt wird, wobei die Phosgenierung eine Quenche umfasst, in der das gebildete gasförmige Verfahrensprodukt enthaltend das herzustellende Isocyanat durch Inkontaktbringen mit einer Quenchflüssigkeit ausgewählt aus der Gruppe bestehend aus Lösungsmittel, dem herzustellenden Isocyanat und Gemischen aus dem herzustellenden Isocyanat und Lösungsmittel, abgekühlt und das herzustellende Isocyanat verflüssigt wird.

4. Verfahren gemäß Anspruch 3, bei welchem die Quenchflüssigkeit ausgewählt ist aus der Gruppe bestehend aus Lösungsmittel und Gemischen aus dem herzustellenden Isocyanat und Lösungsmittel.

5. Verfahren gemäß Anspruch 2 oder 4, bei welchem das Lösungsmittel ausgewählt ist aus der Gruppe bestehend aus Chlorbenzol, ortho-Dichlorbenzol, para-Dichlorbenzol, den Isomeren des Trichlorbenzols, Toluol, den Isomeren des Xylols und Mischungen der vorgenannten Lösungsmittel.

**6.** Verfahren gemäß einem der Ansprüche 2, 4 oder 5, bei welchem die gegebenenfalls vorhandenen Leichtsieder aus Lösungsmittel und Nebenkomponenten bestehen, wobei der Massenanteil an Nebenkomponenten, bezogen auf die Gesamtmasse des Destillationssumpfstromes, maximal 0,10 Massen-% beträgt.

**7.** Verfahren gemäß einem der vorstehenden Ansprüche, umfassend den Schritt 1), wobei die Vorkonzentrierung bei einer Temperatur im Bereich von 120 °C bis 180 °C und bei einem Druck im Bereich von 20 $mbar_{(abs.)}$ bis 60 $mbar_{(abs.)}$ erfolgt.

**8.** Verfahren gemäß einem der vorstehenden Ansprüche, bei welchem als Trocknungsvorrichtung in Schritt 2) ein Apparat ausgewählt aus der Gruppe bestehend aus beheizten, produktumschaufelnden Vakuumtrocknern mit horizontaler Welle, Drehrohren, Scheibentrocknern, Bandtrocknern und Granulierschnecken verwendet wird.

**9.** Verfahren gemäß einen der vorstehenden Ansprüche, umfassend die folgenden Schritte:

a) Phosgenierung des zu dem herzustellenden Isocyanat korrespondierenden primären Amins unter Erhalt eines das herzustellende Isocyanat umfassenden flüssigen rohen Verfahrensprodukts und eines Chlorwasserstoff umfassenden gasförmigen rohen Verfahrensprodukts, wobei optional ein Lösungsmittel eingesetzt wird;
b) Aufarbeitung des in Schritt a) erhaltenen flüssigen rohen Verfahrensprodukts umfassend die Schritte:

b.1) optionale Abtrennung von gelöstem Phosgen und gelöstem Chlorwasserstoff aus dem in Schritt a) erhaltenen flüssigen rohen Verfahrensprodukt unter Erhalt eines an Phosgen und Chlorwasserstoff abgereicherten flüssigen Verfahrensprodukts;
b.2) optionale Abtrennung von Lösungsmittel aus dem in Schritt a) erhaltenen flüssigen rohen Verfahrensprodukt oder aus dem in Schritt b) erhaltenen, an Phosgen und Chlorwasserstoff abgereicherten flüssigen Verfahrensprodukt unter Erhalt eines an Phosgen, Chlorwasserstoff und Lösungsmittel abgereicherten flüssigen Verfahrensprodukts;
b.3) Destillation des in Schritt a) erhaltenen flüssigen rohen Verfahrensprodukts oder des in Schritt b.1) erhaltenen, an Phosgen und Chlorwasserstoff abgereicherten flüssigen Verfahrensprodukts oder des in Schritt b.2) erhaltenen, an Phosgen, Chlorwasserstoff und Lösungsmittel abgereicherten flüssigen Verfahrensprodukts unter Erhalt eines einen ersten Teil des herzustellenden Isocyanats umfassenden Destillatstroms und eines aus Destillationsrückstand, einem zweiten Teil des herzustellenden Isocyanats und gegebenenfalls Leichtsiedern, insbesondere Lösungsmittel bestehenden Destillationssumpfstroms;
wobei der in Schritt b.3) erhaltene Destillationssumpfstrom der in Schritt 2) oder in Schritt 1) und Schritt 2) aufzuarbeitende Destillationssumpfstrom ist.

**10.** Verfahren gemäß Anspruch 9, bei welchem die Einstellung des auf die Gesamtmasse des der Trocknungsvorrichtung zugeführten Destillationsrückstandes bezogene Massenanteil an Carbodiimidgruppen-haltigen Verbindungen über ausschließlich eine, zwei oder alle der folgenden Maßnahmen bewirkt wird:

A. Einstellung von die Bildung von Carbodiimidgruppen-haltigen Verbindungen begünstigenden Verfahrensbedingungen in Schritt a) und/oder in Schritt b) und/oder - sofern durchgeführt - in Schritt 1);
B. Herstellung von Carbodiimidgruppen-haltigen Verbindungen aus dem herzustellen Isocyanat in einem separaten Prozess und Zuführen der so hergestellten Carbodiimidgruppen-haltigen Verbindungen in die Trocknungsvorrichtung aus Schritt 2);
C. *In situ*-Bildung von Carbodiimidgruppen-haltigen Verbindungen in Schritt 2).

**11.** Verfahren gemäß Anspruch 10, bei welchem Maßnahme A umfasst ist, wobei die Einstellung von die Bildung von Carbodiimidgruppen-haltigen Verbindungen begünstigenden Verfahrensbedingungen in Schritt a) und/oder in Schritt b) und/oder - sofern durchgeführt - in Schritt 1) durch eine Erhöhung der Temperatur im jeweiligen Schritt und/oder durch eine Erhöhung der Verweilzeit des den jeweiligen Schritt durchlaufenden Verfahrensprodukts bewirkt wird.

**12.** Verfahren gemäß Anspruch 10, bei welchem Maßnahme B umfasst ist, wobei die Herstellung von Carbodiimidgruppen-haltigen Verbindungen aus dem herzustellen Isocyanat in einem separaten Prozess Folgendes umfasst:

Temperung eines das herzustellende Isocyanat enthaltenden Verfahrensprodukts aus dem erfindungsgemäßen Herstellverfahren
in Abwesenheit von Katalysatoren bei einer Temperatur im Bereich von 200 °C bis 270 °C oder

in Gegenwart von Katalysatoren vom Phospholinoxid bei einer Temperatur im Bereich von 50 °C bis 150 °C.

**13.** Verfahren gemäß Anspruch 11 oder 12, bei dem Schritt 2) bei einem Druck im Bereich von 10 $mbar_{(abs.)}$ bis 250 $mbar_{(abs.)}$, bevorzugt im Bereich von 20 $mbar_{(abs.)}$ bis 200 $mbar_{(abs.)}$, besonders bevorzugt im Bereich von 30 mbar($_{abs}$.) bis 100 mbar($_{abs}$.) durchgeführt wird.

**14.** Verfahren gemäß Anspruch 10, bei welchem Maßnahme C umfasst ist, wobei die Trocknung gemäß Schritt 2) zunächst in einem ersten Teilschritt 2.1) bei einem Druck im Bereich von > 750 mbar($_{abs}$.) bis 1013 $mbar_{(abs.)}$ und bei einer Temperatur im Bereich von 200 °C bis 270 °C und dann in einem zweiten Teilschritt 2.2) bei einem Druck im Bereich von 10 mbar($_{abs}$.) bis 250 $mbar_{(abs.)}$, bevorzugt 20 $mbar_{(abs.)}$ bis 200 $mbar_{(abs.)}$, besonders bevorzugt 30 $mbar_{(abs.)}$ bis 100 mbar($_{abs}$.), und bei einer Temperatur im Bereich 200 °C bis 270 °C durchgeführt wird.
oder
bei einem Druck im Bereich von > 250 mbar($_{abs}$.) bis 750 mbar($_{abs}$.), bevorzugt 300 mbar($_{abs}$.) bis 650 $mbar_{(abs.)}$, besonders bevorzugt 450 $mbar_{(abs.)}$ bis 550 mbar($_{abs}$.) und einer Temperatur im Bereich von 200 °C bis 270 °C durchgeführt wird.

**15.** Verfahren gemäß einem der vorstehenden Ansprüche, bei welchem das herzustellende Isocyanat ausgewählt ist aus der Gruppe bestehend aus Toluylendiisocyanat, Naphthyldiisocyanat, 1,5-Pentandiisocyanat, 1,6-Hexamethylendiisocyanat, Isophorondiisocyanat, Xylylendiisocyanat und Diisocyanatodicyclohexylmethan.

## Claims

**1.** Process for preparing an isocyanate by phosgenating the primary amine corresponding to the isocyanate to be prepared to obtain a liquid crude process product comprising the isocyanate to be prepared,
comprising the workup of said liquid crude process product to obtain a distillation bottom stream consisting of

- the isocyanate to be prepared,
- optionally low boilers, i.e. substances or azeotropically boiling mixtures of substances having a lower boiling point than the isocyanate to be prepared, or, if the isocyanate to be prepared is in the form of an isomer mixture, having a boiling point lower than the lowest-boiling isomer of the isocyanate to be prepared, and
- distillation residue;

further comprising the workup of said distillation bottom stream, said workup comprising the following steps:

1) optionally pre-concentrating the distillation bottom stream in an evaporator by partially evaporating the isocyanate to be prepared which is present in the distillation bottom stream to obtain a pre-concentrated liquid stream depleted of isocyanate to be prepared;
2) drying the distillation bottom stream or the pre-concentrated liquid stream depleted of isocyanate to be prepared which has been obtained in step 1) in a drying apparatus at a temperature in the range from 150°C to 500°C, preferably in the range from 185°C to 300°C, more preferably in the range from 200°C to 270°C, with evaporation and recovery of isocyanate to be prepared to form a solid process product, where the proportion by mass of compounds containing carbodiimide groups based on the total mass of the distillation residue supplied to the drying apparatus is adjusted to a value of at least 15%, preferably at least 20%, more preferably at least 30%.

**2.** Process according to Claim 1, in which the phosgenation is conducted in the liquid phase in the presence of a solvent.

**3.** Process according to Claim 1, in which the phosgenation is conducted in the gas phase, the phosgenation comprising a quench in which the gaseous process product formed, comprising the isocyanate to be prepared, is cooled and the isocyanate to be prepared is liquefied by contacting with a quench liquid selected from the group consisting of solvent, the isocyanate to be prepared and mixtures of the isocyanate to be prepared and solvent.

**4.** Process according to Claim 3, in which the quench liquid is selected from the group consisting of solvent and mixtures of the isocyanate to be prepared and solvent.

**5.** Process according to Claim 2 or 4, in which the solvent is selected from the group consisting of chlorobenzene, ortho-dichlorobenzene, para-dichlorobenzene, the isomers of trichlorobenzene, toluene, the isomers of xylene and

mixtures of the aforementioned solvents.

**6.** Process according to any of Claims 2, 4 and 5, in which any low boilers present consist of solvent and secondary components, where the proportion by mass of secondary components, based on the total mass of the distillation bottom stream, is not more than 0.10% by mass.

**7.** Process according to any of the preceding claims, including step 1), wherein the pre-concentration is effected at a temperature in the range from 120°C to 180°C and a pressure in the range from 20 mbar$_{(abs.)}$ to 60 mbar$_{(abs.)}$ .

**8.** Process according to any of the preceding claims, in which the drying apparatus used in step 2) is an apparatus selected from the group consisting of heated, product-agitating vacuum driers with a horizontal shaft, rotary tubes, disk driers, belt driers and pelletizing screws.

**9.** Process according to any of the preceding claims, comprising the following steps:

a) phosgenating the primary amine corresponding to the isocyanate to be prepared to obtain a liquid crude process product comprising the isocyanate to be prepared and a hydrogen chloride-comprising gaseous crude process product, with optional use of a solvent;
b) working up the liquid crude process product obtained in step a), comprising the steps of:

b.1) optionally separating dissolved phosgene and dissolved hydrogen chloride from the liquid crude process product obtained in step a) to obtain a phosgene- and hydrogen chloride-depleted liquid process product;
b.2) optionally separating solvent from the liquid crude process product obtained in step a) or from the phosgene- and hydrogen chloride-depleted liquid process product obtained in step b) to obtain a phosgene-, hydrogen chloride- and solvent-depleted liquid process product;
b.3) distilling the liquid crude process product obtained in step a) or the phosgene- and hydrogen chloride-depleted liquid process product obtained in step b.1) or the phosgene-, hydrogen chloride-and solvent-depleted liquid process product obtained in step b.2) to obtain a distillate stream comprising a first portion of the isocyanate to be prepared and a distillate bottom stream consisting of distillation residue, a second portion of the isocyanate to be prepared and optionally low boilers, especially solvent;
wherein the distillation bottom stream obtained in step b.3) is the distillation bottom stream to be worked up in step 2) or in step 1) and step 2).

**10.** Process according to Claim 9, in which the adjustment of the proportion by mass of compounds containing carbodiimide groups based on the total mass of the distillation residue supplied to the drying apparatus is brought about via exclusively one, two or all of the following measures:

A. establishing process conditions that promote the formation of compounds containing carbodiimide groups in step a) and/or in step b) and/or - if conducted - in step 1);
B. preparing compounds containing carbodiimide groups from the isocyanate to be prepared in a separate operation and supplying the compounds containing carbodiimide groups thus prepared to the drying apparatus from step 2);
C. forming compounds containing carbodiimide groups *in situ* in step 2).

**11.** Process according to Claim 10, including measure A, wherein the establishment of process conditions that promote the formation of compounds containing carbodiimide groups in step a) and/or in step b) and/or - if conducted - in step 1) is brought about by an increase in the temperature in the respective step and/or by an increase in the residence time of the process product that passes through the respective step.

**12.** Process according to Claim 10, including measure B, wherein the preparation of compounds containing carbodiimide groups from the isocyanate to be prepared in a separate operation comprises the following:

heating a process product containing the isocyanate to be prepared from the preparation process of the invention in the absence of catalysts at a temperature in the range from 200°C to 270°C
or
in the presence of catalysts of the phospholine oxide at a temperature in the range from 50°C to 150°C.

**13.** Process according to Claim 11 or 12, in which step 2) is conducted at a pressure in the range from 10 mbar$_{(abs.)}$

to 250 mbar($_{abs}$.), preferably in the range from 20 mbar($_{abs}$.) to 200 mbar($_{abs}$.), more preferably in the range from 30 mbar$_{(abs.)}$ to 100 mbar$_{(abs.)}$.

**14.** Process according to Claim 10, including measure C, wherein the drying in step 2) is conducted first in a first partial step 2.1) at a pressure in the range from > 750 mbar($_{abs}$.) to 1013 mbar($_{abs}$.) and at a temperature in the range from 200°C to 270°C and then in a second partial step 2.2) at a pressure in the range from 10 mbar$_{(abs.)}$ to 250 mbar$_{(abs.)}$, preferably 20 mbar($_{abs}$.) to 200 mbar($_{abs}$.), more preferably 30 mbar($_{abs}$.) to 100 mbar$_{(abs.)}$, and at a temperature in the range from 200°C to 270°C,
or
at a pressure in the range from > 250 mbar($_{abs}$.) to 750 mbar$_{(abs.)}$, preferably 300 mbar$_{(abs.)}$ to 650 mbar($_{abs}$.), more preferably 450 mbar($_{abs}$.) to 550 mbar($_{abs}$.), and a temperature in the range from 200°C to 270°C.

**15.** Process according to any of the preceding claims, in which the isocyanate to be prepared is selected from the group consisting of tolylene diisocyanate, naphthyl diisocyanate, pentane 1,5-diisocyanate, hexamethylene 1,6-diisocyanate, isophorone diisocyanate, xylylene diisocyanate and diisocyanatodicyclohexylmethane.

**Revendications**

**1.** Procédé de fabrication d'un isocyanate par phosgénation de l'amine primaire correspondant à l'isocyanate à fabriquer avec obtention d'un produit de procédé brut liquide comprenant l'isocyanate à fabriquer,
comprenant le traitement de ce produit de procédé brut liquide avec obtention d'un courant de fond de distillation constitué par

- l'isocyanate à fabriquer,
- éventuellement des composants de point d'ébullition faible, qui sont des substances ou des mélanges à ébullition azéotropique de substances, dont le point d'ébullition est inférieur à celui de l'isocyanate à fabriquer, ou, si l'isocyanate à fabriquer se présente sous la forme d'un mélange d'isomères, est inférieur à celui de l'isomère ayant le point d'ébullition le plus bas de l'isocyanate à fabriquer, et
- un résidu de distillation ;

comprenant en outre le traitement de ce courant de fond de distillation, ce traitement comprenant les étapes suivantes :

1) la préconcentration éventuelle du courant de fond de distillation dans un évaporateur par évaporation partielle de l'isocyanate à fabriquer contenu dans le courant de fond de distillation, un courant liquide préconcentré appauvri en isocyanate à fabriquer étant obtenu ;
2) le séchage du courant de fond de distillation ou du courant liquide, appauvri en isocyanate à fabriquer, préconcentré obtenu dans l'étape 1) dans un dispositif de séchage à une température dans la plage allant de 150 °C à 500 °C, de préférence dans la plage allant de 185 °C à 300 °C, de manière particulièrement préférée dans la plage allant de 200 °C à 270 °C, l'isocyanate à fabriquer étant évaporé avec formation d'un produit de procédé solide et récupéré, la proportion en masse de composés contenant des groupes carbodiimide relative à la masse totale du résidu de distillation introduit dans le dispositif de séchage étant ajustée à une valeur d'au moins 15 %, de préférence d'au moins 20 %, de manière particulièrement préférée d'au moins 30 %.

**2.** Procédé selon la revendication 1, selon lequel la phosgénation est réalisée dans la phase liquide en présence d'un solvant.

**3.** Procédé selon la revendication 1, selon lequel la phosgénation est réalisée dans la phase gazeuse, la phosgénation comprenant une trempe, dans laquelle le produit de procédé gazeux formé contenant l'isocyanate à fabriquer est refroidi par mise en contact avec un liquide de trempe choisi dans le groupe constitué par du solvant, l'isocyanate à fabriquer et des mélanges de l'isocyanate à fabriquer et de solvant, et l'isocyanate à fabriquer est liquéfié.

**4.** Procédé selon la revendication 3, selon lequel le liquide de trempe est choisi dans le groupe constitué par du solvant et des mélanges de l'isocyanate à fabriquer et de solvant.

**5.** Procédé selon la revendication 2 ou 4, selon lequel le solvant est choisi dans le groupe constitué par le chlorobenzène, l'ortho-dichlorobenzène, le para-dichlorobenzène les isomères du trichlorobenzène, le toluène, les isomères de

xylène et des mélanges des solvants susmentionnés.

**6.** Procédé selon l'une quelconque des revendications 2, 4 ou 5, selon lequel les composants de point d'ébullition faible éventuellement présents sont constitués par du solvant et des composants secondaires, la proportion en masse de composants secondaires, par rapport à la masse totale du courant de fond de distillation, étant d'au plus 0,10 % en masse.

**7.** Procédé selon l'une quelconque des revendications précédentes, comprenant l'étape 1), la préconcentration ayant lieu à une température dans la plage allant de 120 °C à 180 °C et à une pression dans la plage allant de 20 mbar($_{abs.}$) à 60 mbar($_{abs.}$).

**8.** Procédé selon l'une quelconque des revendications précédentes, selon lequel un appareil choisi dans le groupe constitué par les séchoirs sous vide chauffés brassant le produit à arbre horizontal, les tubes rotatifs, les séchoirs à disque, les séchoirs à bande et les vis de granulation est utilisé en tant que dispositif de séchage dans l'étape 2).

**9.** Procédé selon l'une quelconque des revendications précédentes, comprenant les étapes suivantes :

a) la phosgénation de l'amine primaire correspondant à l'isocyanate à fabriquer avec obtention d'un produit de procédé brut liquide comprenant l'isocyanate à fabriquer et d'un produit de procédé brut gazeux comprenant du chlorure d'hydrogène, un solvant étant éventuellement utilisé ;
b) le traitement du produit de procédé brut liquide obtenu dans l'étape a) comprenant les étapes suivantes :

b.1) la séparation éventuelle de phosgène dissous et de chlorure d'hydrogène dissous du produit de procédé brut liquide obtenu dans l'étape a) avec obtention d'un produit de procédé liquide appauvri en phosgène et chlorure d'hydrogène ;
b.2) la séparation éventuelle de solvant du produit de procédé brut liquide obtenu dans l'étape a) ou du produit de procédé liquide appauvri en phosgène et chlorure d'hydrogène, obtenu dans l'étape b), avec obtention d'un produit de procédé liquide appauvri en phosgène, chlorure d'hydrogène et solvant ;
b.3) la distillation du produit de procédé brut liquide obtenu dans l'étape a) ou du produit de procédé liquide appauvri en phosgène et chlorure d'hydrogène, obtenu dans l'étape b.1), ou du produit de procédé liquide appauvri en phosgène, chlorure d'hydrogène et solvant, obtenu dans l'étape b.2), avec obtention d'un courant de distillat comprenant une première partie de l'isocyanate à fabriquer et d'un courant de fond de distillation constitué par un résidu de distillation, une deuxième partie de l'isocyanate à fabriquer et éventuellement des composants de point d'ébullition faible, notamment du solvant ;
le courant de fond de distillation obtenu dans l'étape b.3) étant le courant de fond de distillation à traiter dans l'étape 2) ou dans l'étape 1) et l'étape 2).

**10.** Procédé selon la revendication 9, selon lequel l'ajustement de la proportion en masse de composés contenant des groupes carbodiimide relative à la masse totale du résidu de distillation introduit dans le dispositif de séchage est effectué par le biais d'exclusivement une, de deux ou de toutes les mesures suivantes :

A. l'ajustement de conditions de procédé favorisant la formation de composés contenant des groupes carbodiimide dans l'étape a) et/ou dans l'étape b) et/ou - si elle est réalisée - dans l'étape 1) ;
B. la fabrication de composés contenant des groupes carbodiimide à partir de l'isocyanate à fabriquer dans un procédé séparé et l'introduction des composés contenant des groupes carbodiimide ainsi fabriqués dans le dispositif de séchage de l'étape 2) ;
C. la formation *in situ* de composés contenant des groupes carbodiimide dans l'étape 2).

**11.** Procédé selon la revendication 10, selon lequel la mesure A est comprise, l'ajustement de conditions de procédé favorisant la formation de composés contenant des groupes carbodiimide dans l'étape a) et/ou dans l'étape b) et/ou - si elle est réalisée - dans l'étape 1) étant effectué par une augmentation de la température dans l'étape en question et/ou par une augmentation du temps de séjour du produit de procédé traversant l'étape en question.

**12.** Procédé selon la revendication 10, selon lequel la mesure B est comprise, la fabrication de composés contenant des groupes carbodiimide à partir de l'isocyanate à fabriquer dans un procédé séparé comprenant les étapes suivantes :

le recuit d'un produit de procédé contenant l'isocyanate à fabriquer à partir du procédé de fabrication selon

l'invention
en l'absence de catalyseurs à une température dans la plage allant de 200 °C à 270 °C
ou
en présence de catalyseurs de l'oxyde de phospholine à une température dans la plage allant de 50 °C à 150 °C.

**13.** Procédé selon la revendication 11 ou 12, selon lequel l'étape 2) est réalisée à une pression dans la plage allant de 10 mbar$_{(abs.)}$ à 250 mbar$_{(abs.)}$, de préférence dans la plage allant de 20 mbar$_{(abs.)}$ à 200 mbar$_{(abs.)}$, de manière particulièrement préférée dans la plage allant de 30 mbar$_{(abs.)}$ à 100 mbar$_{(abs.)}$.

**14.** Procédé selon la revendication 10, selon lequel la mesure C est comprise, le séchage selon l'étape 2)
étant réalisé tout d'abord dans une première étape partielle 2.1) à une pression dans la plage allant de > 750 mbar$_{(abs.)}$ à 1 013 mbar$_{(abs.)}$ et à une température dans la plage allant de 200 °C à 270 °C, et ensuite dans une deuxième étape partielle 2.2) à une pression dans la plage allant de 10 mbar$_{(abs.)}$ à 250 mbar$_{(abs.)}$, de préférence de 20 mbar$_{(abs.)}$ à 200 mbar$_{(abs.)}$, de manière particulièrement préférée de 30 mbar$_{(abs.)}$ à 100 mbar$_{(abs.)}$, et à une température dans la plage allant de 200 °C à 270 °C,
ou
étant réalisée à une pression dans la plage allant de > 250 mbar$_{(abs.)}$ à 750 mbar$_{(abs.)}$, de préférence de 300 mbar$_{(abs.)}$ à 650 mbar$_{(abs.)}$, de manière particulièrement préférée de 450 mbar$_{(abs.)}$ à 550 mbar$_{(abs.)}$ et à une température dans la plage allant de 200 °C à 270 °C.

**15.** Procédé selon l'une quelconque des revendications précédentes, selon lequel l'isocyanate à fabriquer est choisi dans le groupe constitué par le diisocyanate de toluylène, le diisocyanate de naphtyle, le diisocyanate de 1,5-pentane, le diisocyanate de 1,6-hexaméthylène, le diisocyanate d'isophorone, le diisocyanate de xylylène et le diisocyanatodicyclohexylméthane.

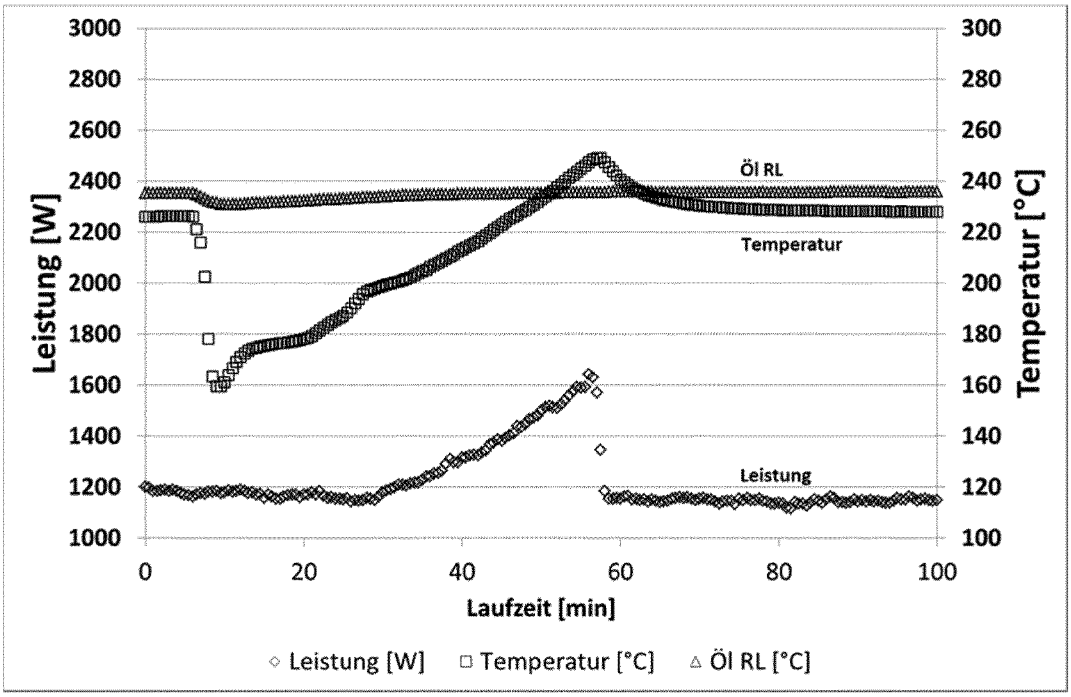
3000
2800
2600
2400
2200
2000
1800
1600
1400
1200
1000
Leistung [W]
300
280
260
240
220
200
180
160
140
120
100
Temperatur [°C]
Öl RL
Temperatur
Leistung
0
20
40
60
80
100
Laufzeit [min]
Leistung [W]
Temperatur [°C]
Öl RL [°C]

FIG. 1

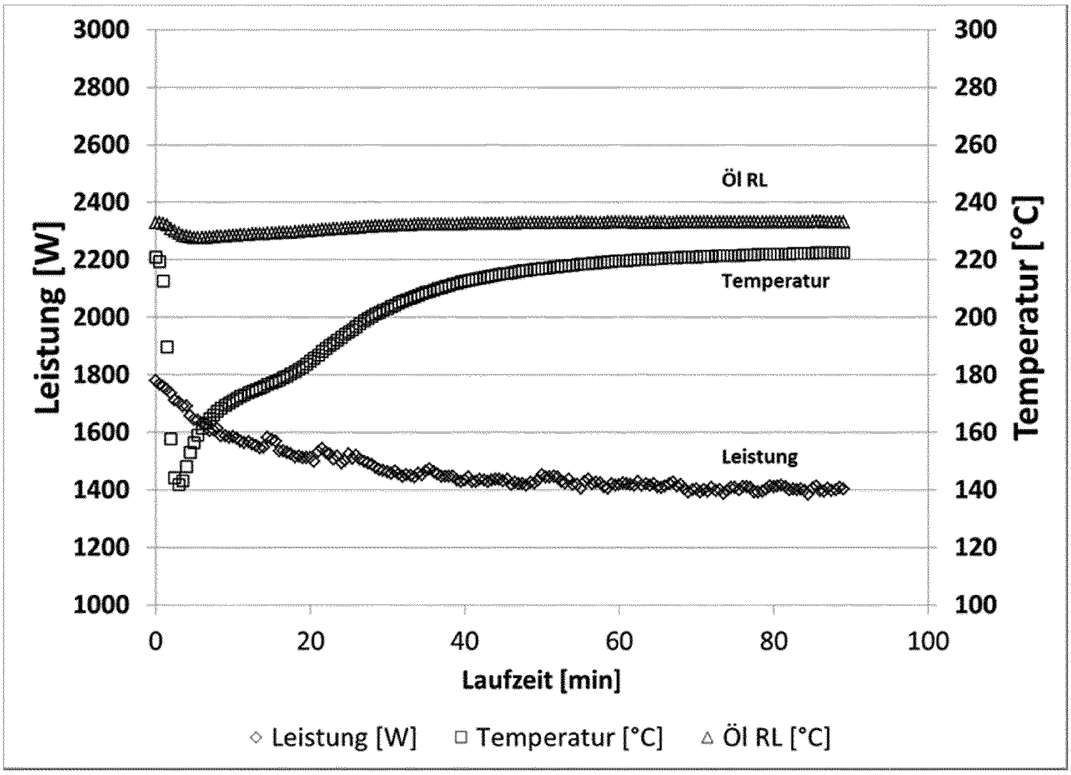
3000
2800
2600
2400
2200
2000
1800
1600
1400
1200
1000
Leistung [W]
300
280
260
240
220
200
180
160
140
120
100
Temperatur [°C]
Öl RL
Temperatur
Leistung
0
20
40
60
80
100
Laufzeit [min]
Leistung [W]
Temperatur [°C]
Öl RL [°C]

FIG. 2

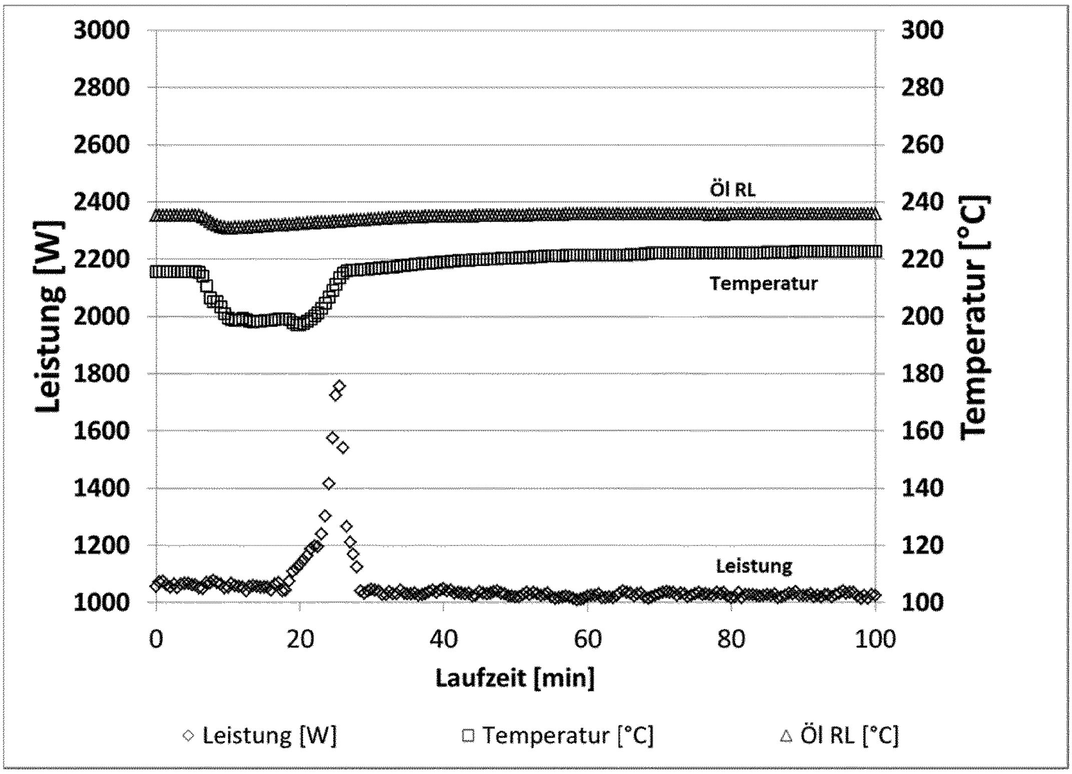
3000
2800
2600
2400
2200
2000
1800
1600
1400
1200
1000
Leistung [W]
300
280
260
240
220
200
180
160
140
120
100
Temperatur [°C]
Öl RL
Temperatur
Leistung
0
20
40
60
80
100
Laufzeit [min]
Leistung [W]
Temperatur [°C]
Öl RL [°C]

FIG. 3

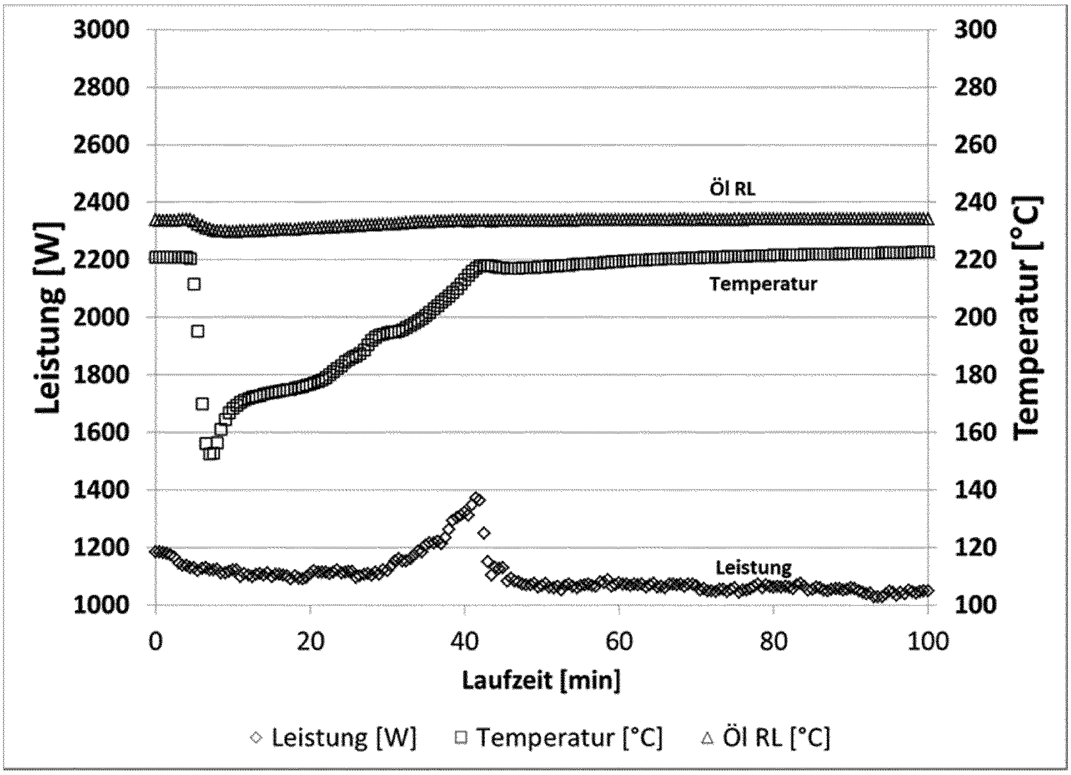
3000
2800
2600
2400
2200
2000
1800
1600
1400
1200
1000
Leistung [W]
300
280
260
240
220
200
180
160
140
120
100
Temperatur [°C]
Öl RL
Temperatur
Leistung
0
20
40
60
80
100
Laufzeit [min]
Leistung [W]
Temperatur [°C]
Öl RL [°C]

FIG. 4

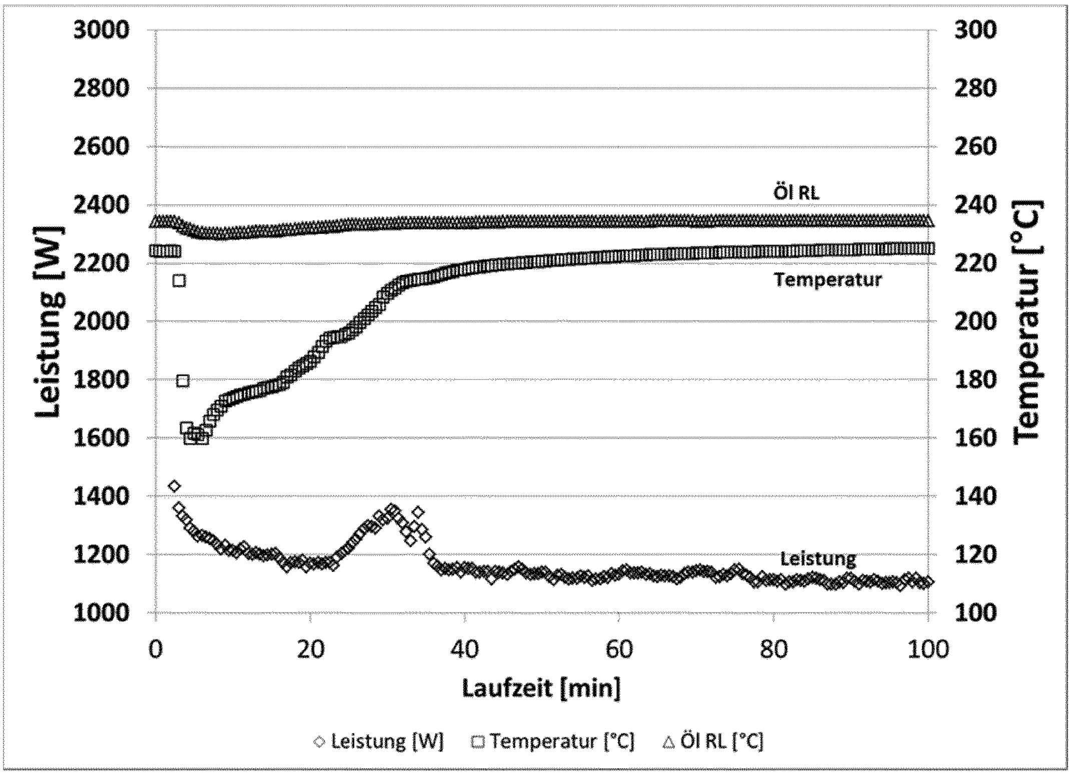

3000
2800
2600
2400
2200
2000
1800
1600
1400
1200
1000
Leistung [W]
300
280
260
240
220
200
180
160
140
120
100
Temperatur [°C]
Öl RL
Temperatur
Leistung
0
20
40
60
80
100
Laufzeit [min]
◇ Leistung [W]
□ Temperatur [°C]
△ Öl RL [°C]


FIG. 5

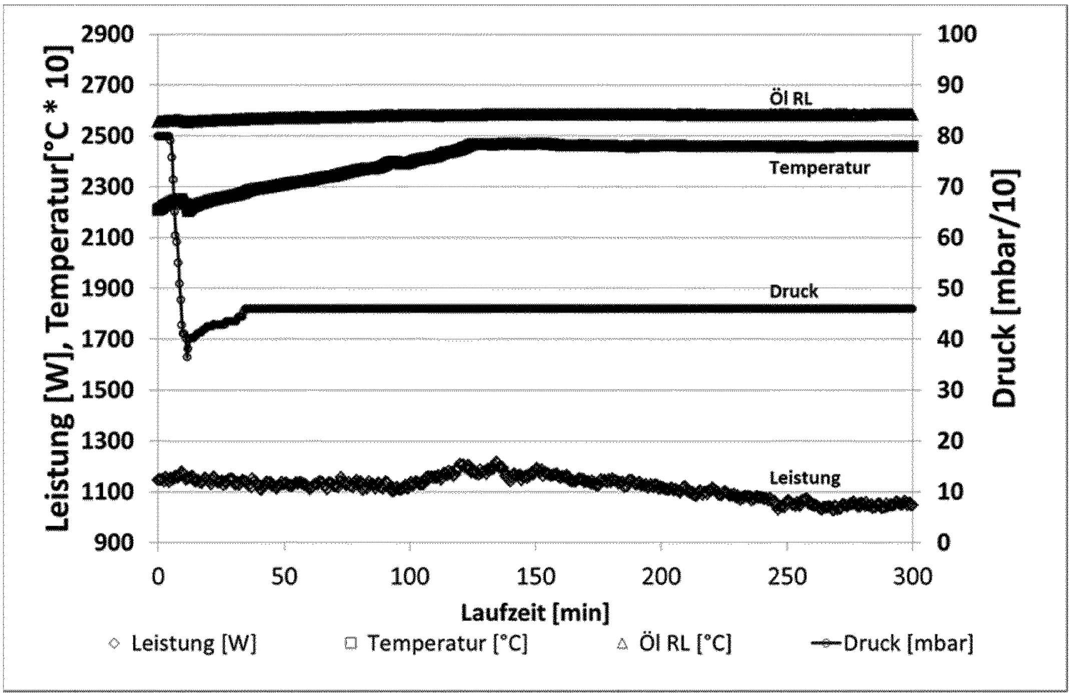

2900
2700
2500
2300
2100
1900
1700
1500
1300
1100
900
Leistung [W], Temperatur[°C * 10]
100
90
80
70
60
50
40
30
20
10
0
Druck [mbar/10]
Öl RL
Temperatur
Druck
Leistung
0
50
100
150
200
250
300
Laufzeit [min]
◇ Leistung [W]
□ Temperatur [°C]
△ Öl RL [°C]
Druck [mbar]


FIG. 6

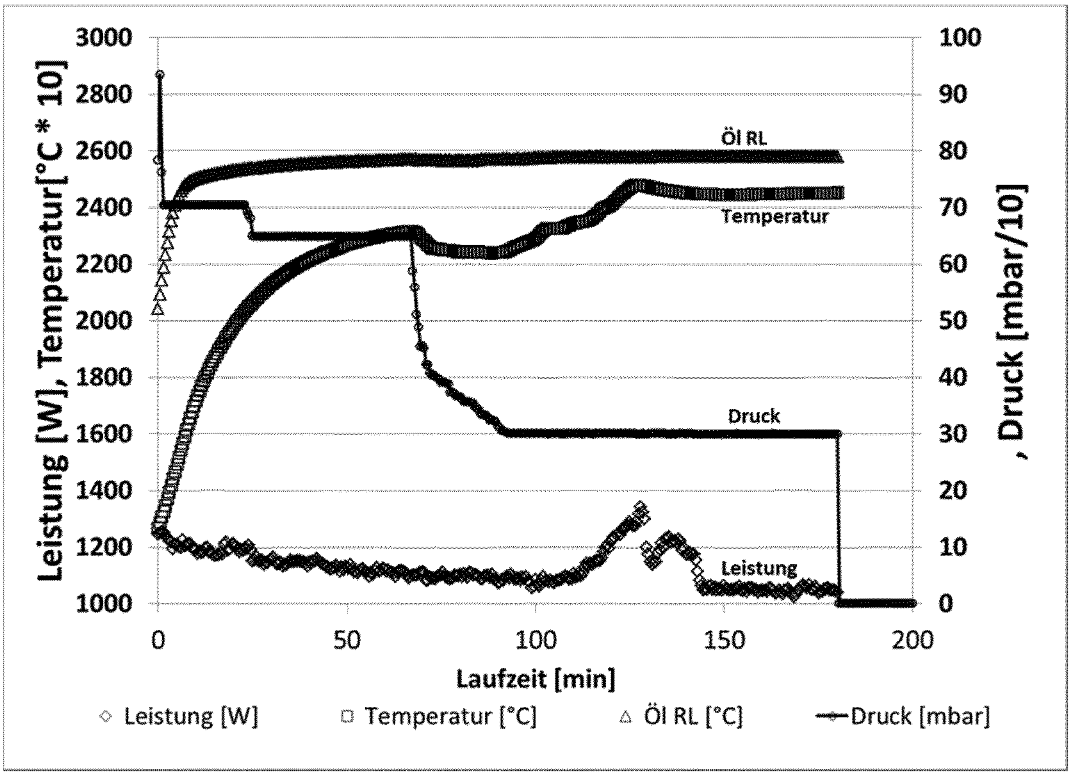

3000
2800
2600
2400
2200
2000
1800
1600
1400
1200
1000
Leistung [W], Temperatur[°C * 10]
100
90
80
70
60
50
40
30
20
10
0
, Druck [mbar/10]
0
50
100
150
200
Laufzeit [min]
Öl RL
Temperatur
Druck
Leistung
◇ Leistung [W]
□ Temperatur [°C]
△ Öl RL [°C]
Druck [mbar]


FIG. 7

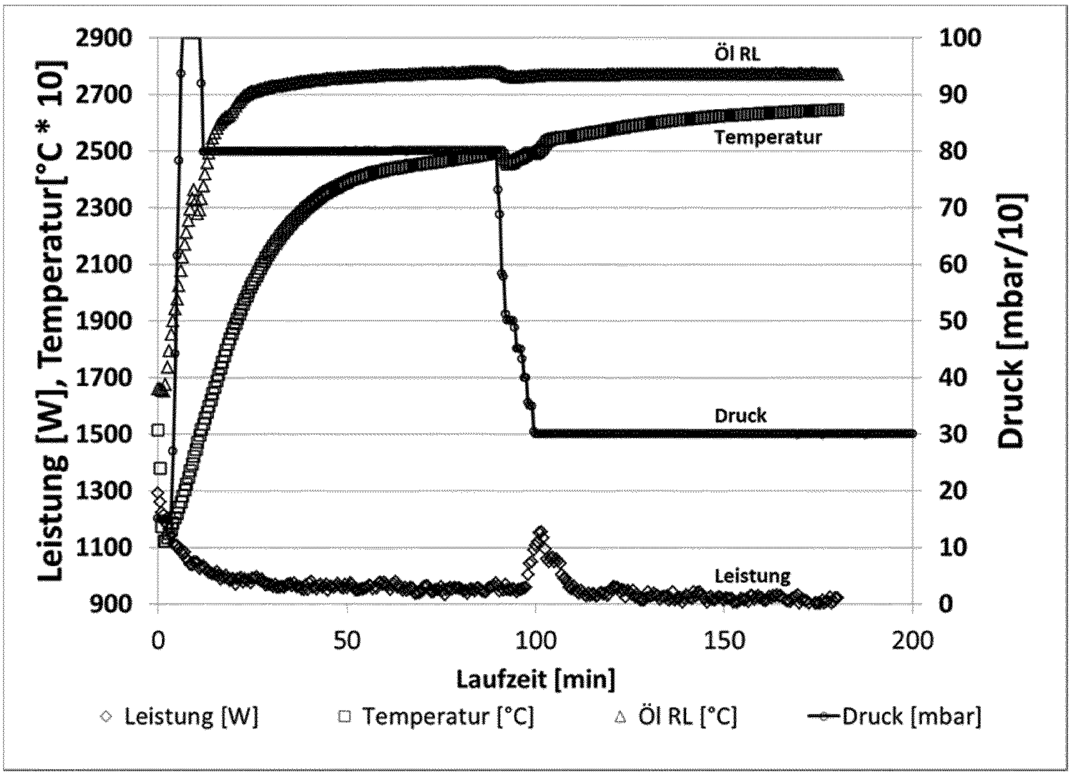

2900
2700
2500
2300
2100
1900
1700
1500
1300
1100
900
Leistung [W], Temperatur[°C * 10]
100
90
80
70
60
50
40
30
20
10
0
Druck [mbar/10]
0
50
100
150
200
Laufzeit [min]
Öl RL
Temperatur
Druck
Leistung
◇ Leistung [W]
□ Temperatur [°C]
△ Öl RL [°C]
Druck [mbar]


FIG. 8

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5314588 A
- US 2884360 A
- US 2884362 A
- US 3987075 A
- US 4372891 A
- DE 4104305 A1
- US 6307096 B
- US 4918220 A
- DD 288599
- EP 0548685 A2
- EP 0626368 A1
- US 5902459 A
- DE 4211774 A1
- US 3694323 A
- US 3128310 A
- US 3331876 A
- GB 795639 A
- DE 2703313 A1
- EP 1935877 A1
- EP 1413571 A1
- EP 1371633 A1
- EP 0017972 A1
- WO 2014009342 A1
- EP 1369412 A1
- EP 1754698 B1
- EP 0289840 B1
- DE 3744001 C1
- EP 0314985 A1
- DE 10260027 A
- DE 10260093 A
- DE 10310888 A
- DE 102006022448 A
- US 20070299279 A
- DE 1792660 A
- US 4289732 A
- US 4419295 A
- US 3713833 A
- EP 0291819 A
- EP 0291820 A
- EP 0830894 A
- EP 0570799 A1
- EP 1555258 A1
- EP 1526129 A1
- DE 10161384 A1
- EP 1319655 B1
- EP 1362847 B1
- EP 2199277 B1
- EP 1449826 B1
- EP 1526129 B1
- EP 1555258 B1
- EP 1403248 A1
- EP 1935875 A1
- EP 1413571 A
- US 20030230476 A1
- EP 1717223 A2
- EP 0515933 A
- US 6120699 A


### In der Beschreibung aufgeführte Nicht-Patentliteratur


- Ullmann's Encyclopedia of Industrial Chemistry. VCH Verlagsgesellschaft mbH, 1991, vol. A 19, 390
- Polyurethane Handbook. Hanser Verlag, 1993, 60
- **G. WEGENER.** Applied Catalysis A: General. Elsevier Science B.V, 2001, vol. 221, 303-335
- Chem Systems, Process Evaluation Research Planning TDI/MDI 98/99 S8. *Chem System's PERP Report for TDI/MDI,* 1999, 27-32